# EUROPÄISCHE PATENTSCHRIFT

(11) **EP 1 077 669 B1**
(45) Veröffentlichungstag und Bekanntmachung des Hinweises auf die Patenterteilung: **08.09.2004**
(21) Anmeldenummer: 99923589.8
(22) Anmeldetag: 12.05.1999
(51) Int. Cl.: A61K 7/06, A61K 7/48, A61K 47/34, D06N 3/14, D21H 17/57, C09D 175/04

(54) **KOSMETISCHES MITTEL**
COSMETIC PRODUCT
PRODUIT COSMETIQUE

(30) Priorität: 14.05.1998 DE 19821731
(43) Veröffentlichungstag der Anmeldung: 28.02.2001
(73) Patentinhaber: BASF AKTIENGESELLSCHAFT, 67056 Ludwigshafen (DE)
(72) Erfinder: NGUYEN KIM, Son, D-69502 Hemsbach (DE); SANNER, Axel, D-67227 Frankenthal (DE); SCHEHLMANN, Volker, D-67354 Römerberg (DE)
(74) Vertreter: Kinzebach, Werner, Dr.
(86) Internationale Anmeldenummer: PCT/EP1999/003295
(87) Internationale Veröffentlichungsnummer: WO 1999/058100

(56) Entgegenhaltungen:
- EP-A- 0 163 214
- EP-A- 0 773 246
- WO-A-97/17052
- WO-A-97/17386
- DATABASE WPI Section Ch, Week 9349 Derwent Publications Ltd., London, GB; Class A14, AN 93-392731 XP002114927 & JP 05 295078 A (DAINIPPON INK & CHEM KK) , 9. November 1993 (1993-11-09)

## Beschreibung

Die vorliegende Erfindung betrifft ein kosmetisches Mittel, welches wenigstens ein vernetztes, wasserlösliches oder wasserdispergierbares Polyurethan aus mindenstens einem Polyurethanpräpolymer mit endständigen Isocyanatgruppen und mindestens einem Polymerisat mit gegenüber Isocyanatgruppen reaktiven Gruppen enthält.

In der Kosmetik werden Polymere mit filmbildenden Eigenschaften zur Festigung, Strukturverbesserung und Formgebung der Haare verwendet. Diese Haarbehandlungsmittel enthalten im Allgemeinen eine Lösung des Filmbildners in einem Alkohol oder einem Gemisch aus Alkohol und Wasser.

Haarfestigungsmittel werden im Allgemeinen in Form von wässrig-alkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, dass sie aus dem Haar ausgewaschen werden können, andererseits aber sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht und eine relativ hohe Glastemperatur (mindestens 15°C) besitzen.

Bei der Formulierung von Haarfestigern ist außerdem zu berücksichtigen, dass aufgrund der Umweltbestimmungen zur Kontrolle der Emission flüchtiger organischer Verbindungen (VOC = volatile organic compounds) in die Atmosphäre eine Verringerung des Alkoholund Treibmittelgehalts erforderlich ist.

Ein weiterer aktueller Anspruch an Haarbehandlungsmittel ist es, dem Haar ein natürliches Aussehen und Glanz zu verleihen, z. B. auch dann, wenn es sich um von Natur aus besonders kräftiges und/oder dunkles Haar handelt.

Es ist bekannt, Polysiloxane, wie z. B. Polydimethylsiloxan, und Polysiloxanderivate in Haarpflegemitteln einzusetzen.

Die EP-A-017 122 beschreibt die Verwendung von Polysiloxan-Ammoniumderivaten in Haarwasch- und Haarbehandlungsmitteln zur Verbesserung der Kämmbarkeit, Weichheit und Fülle des Haares.

Die EP-A-729 742 beschreibt Haarbehandlungsmittel auf Basis A) eines Amino-modifizierten Siliconterpolymers und B) mindestens eines kationischen, siliconfreien Konditionierungsmittels auf Basis eines quaternären Ammoniumsalzes.

Nachteilig an der in den beiden zuvor genannten Publikationen beschriebenen Verwendung von Polysiloxanen, die nicht kovalent an das Festigerpolymer gebunden sind, ist es, dass häufig Entmischungen der Formulierungen während der Lagerung und nach deren Anwendung auf dem Haar auftreten.

Die EP-A-408 311 beschreibt die Verwendung von Copolymeren mit Einheiten aus a) ethylenisch ungesättigten, hydrophilen Monomeren und b) ethylenisch ungesättigten Monomeren mit Polysiloxangruppen in Haarpflegeprodukten.

Die EP-A-412 704 beschreibt ein Haarpflegemittel auf Basis eines Pfropfcopolymers, welches monovalente Siloxan-Polymereinheiten an einem Rückgrat auf Basis eines Vinylpolymers aufweist. Nach dem Trocknen zerfällt das Polymer in eine diskontinuierliche, siliconhaltige Phase und eine kontinuierliche, siliconfreie Phase.

Die WO 93/03703 beschreibt eine Haarsprayzusammensetzung, umfassend: a) 0,1 bis 2 Gew.-% eines oberflächenaktiven Mittels, b) 0,5 bis 15 Gew.-% eines ionischen Harzes mit einem zahlenmittleren Molekulargewicht von mindestens 300 000 und c) einen flüssigen Träger. Dabei umfasst das ionische Harz siliconhaltige Monomere, wobei das Harz nach dem Trocknen in eine diskontinuierliche, siliconhaltige Phase und eine siliconfreie kontinuierliche Phase zerfällt.

Die EP-A-362 860 beschreibt Alkohol-modifizierte Siliconesterderivate und kosmetische Zusammensetzungen, die diese enthalten.

Keine dieser Publikationen beschreibt Festigerpolymere auf Basis von Polyurethanen mit einer kovalenten Bindung der Siloxangruppen an die Festigerpolymere über stickstoffhaltige Gruppen. Insbesondere sind keine vernetzten, wasserlöslichen oder wasserdispergierbaren Polyurethane beschrieben, die Siloxangruppen enthalten. Des Weiteren eignen sich diese Polymere nur sehr eingeschränkt zur Herstellung VOC-armer Formulierungen.

Es ist bekannt, in Wasser lösliche oder dispergierbare Polyurethane aufgrund ihrer filmbildenden Eigenschaften und einer im Allgemeinen niedrigen Viskosität in Wasser/Ethanol in der Kosmetik einzusetzen. So beschreibt die US-A-4,743,673 hydrophile Polyurethanpolymere mit Carboxygruppen im Polymerrückgrat. Zur Erzeugung dieser Carboxygruppen werden an das Polymerrückgrat gebundene Estergruppen durch 30- bis 60-minütiges Erhitzen mit einer starken Base verseift. Dabei erfolgt jedoch nicht nur eine Verseifung der Estergruppen der Carbonsäureesterkomponente, sondern auch eine Verseifung der in der Polyurethankette enthaltenen Estergruppierungen. Es kommt somit zur Spaltung der Polyurethankette und zu einer drastischen Verringerung des Molekulargewichts der Polyurethane. Eine Anwendung der Polyurethane in Haarsprays ist zwar erwähnt. In der Praxis sind die mit diesen Polyurethanen erhaltenen Filme für die Haarkosmetik aber nicht brauchbar, weil sie entweder wasserunlöslich sind oder ein zu geringes Molekulargewicht und somit unzureichende Festigungswirkung besitzen.

Die DE-A-42 25 045 und die WO 94/03515 beschreiben die Verwendung von wasserlöslichen oder in Wasser dispergierbaren, anionischen Polyurethanen als Haarfestiger. Diese Polyurethane sind aufgebaut aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat.

Haarfestigerpolymere auf Basis dieser Polyurethane sind im Hinblick auf die Elastizität verbesserungswürdig und weisen im Allgemeinen ohne den Zusatz von Additiven keinen angenehmen Griff auf.

Die DE-A-42 41 118 beschreibt die Verwendung von unvernetzten kationischen Polyurethanen und Polyharnstoffen als Hilfsmittel in kosmetischen und pharmazeutischen Zubereitungen.

Die EP-A-619 111 beschreibt die Verwendung von Polyurethanen auf Basis von organischen Diisocyanaten, Diolen und 2,2-Hydroxymethyl-substituierten Carboxylaten der Formel 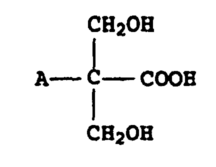 worin A für ein Wasserstoffatom oder eine C₁-C₂₀-Alkylgruppe steht, in Haarfixierungsmitteln. Zumindest ein Teil der Carbonsäuregruppen wird dabei mit einer organischen oder anorganischen Base neutralisiert. Filme auf Basis dieser Polyurethane sind weich und klebrig und die darauf basierenden Haarfestiger entsprechend verbesserungswürdig.

Die in den zuletzt erwähnten Publikationen beschriebenen Polyurethane können die Anforderungen an Haarfestigerpolymere nur teilweise erfüllen. So ist die erwünschte Geschmeidigkeit des Haares bei allen zuvor genannten Produkten auf Polyurethanbasis verbesserungswürdig.

Die EP-A-636 361 beschreibt eine kosmetische Zusammensetzung, welche in einem kosmetisch verträglichen Träger mindestens einen Pseudolatex auf Basis eines Polykondensates umfasst, das mindestens eine Polysiloxaneinheit und mindestens eine Polyurethanund/oder Polyharnstoffeinheit mit anionischen oder kationischen Gruppen umfasst. Vernetzte, wasserlösliche oder wasserdispergierbare Polyurethane sind nicht beschrieben. Die WO 97/25021 hat einen vergleichbaren Offenbarungsgehalt. Diese kosmetischen Mittel eignen sich unter anderem zur Behandlung von keratinischen Materialien. Die Auswaschbarkeit dieser Filmbildner ist jedoch nicht zufriedenstellend. Zudem besitzen sie aufgrund eines hohen Siloxananteils auch nicht die für ein Haarpolymer erforderliche Festigungswirkung.

Die DE-A-195 41 329 und die WO 97/17052 beschreiben Haarbehandlungsmittel, enthaltend ein in Wasser lösliches oder dispergierbares Salz der Formel I:

[A-(X)ₙ]ⁿ⁻ • [HₘB]^{m+} I

worin
- A: für einen kosmetisch akzeptablen aliphatischen, cycloaliphatischen oder aromatischen Rest steht, der siloxan- und/oder fluorhaltige Einheiten aufweisen kann,
- X: für eine Carboxylat-, Sulfonat-, Phosphat- oder Phosphonatgruppe steht;
- B: eine kosmetisch akzeptable Aminbase bedeutet die siloxan-und/oder fluorhaltige Einheiten aufweisen kann;
- n: für 1 bis 30 steht; und
- m: die Wertigkeit des Amins B bedeutet.

Haarspray-Formulierungen auf Basis dieser siloxanhaltigen Salze, einem nicht siloxanhaltigen Haarfestigerpolymer und einem Siliconöl führen zu Filmen, die leicht, z. B. durch mechanische Belastung, von der Haaroberfläche abgelöst werden. Die Festigungswirkung dieser Formulierungen ist daher verbesserungswürdig.

Die DE-A-195 41 326 und die WO 97/17386 beschreiben wasserlösliche oder wasserdispergierbare Polyurethane mit endständigen Säuregruppen, ihre Herstellung und ihre Verwendung. Dabei wird ein in Wasser lösliches oder dispergierbares Polyurethanpräpolymer mit endständigen Isocyanatgruppen mit einer Aminosulfonsäure oder Aminocarbonsäure, insbesondere Taurin, Asparaginsäure und Glutaminsäure, umgesetzt. Haarsprays auf Basis dieser Polyurethane sind noch verbesserungswürdig. Insbesondere bei der Formulierung von Haarsprays mit einem hohen Treibgasgehalt und/oder einem hohen Gehalt an organischen Lösungsmitteln und gegebenenfalls gleichzeitigem Einsatz von Sprühzerstäubern zur Erzielung sehr kleiner Tröpfchen können Probleme auftreten.

Die EP-A-492 657 beschreibt ein kosmetischen Mittel zur Verwendung in Haut- und Haarpflegeprodukten, welches ein lineares Polysiloxan-Polyoxyalkylen-Blockcopolymer als Wiederholungseinheit enthält. Vernetzte, wasserlösliche oder wasserdispergierbare Polyurethane auf Basis eines siloxanhaltigen oder siloxanfreien Polyurethanpräpolymers sind in diesem Dokument nicht beschrieben.

Die EP-A-277 816 beschreibt Polydimethylsiloxane mit zwei Hydroxylgruppen an einem Kettenende und einer Trimethylsilylgruppe am anderen, der allgemeinen Formel 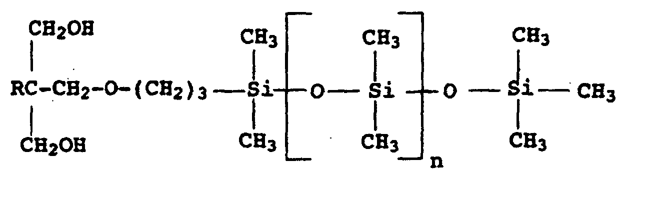 worin R für Wasserstoff, Methyl oder Ethyl steht und n für einen Wert von 0 bis 4 000 steht, sowie damit modifizierte Polyurethane. Dabei handelt es sich ausschließlich um modifizierte Polyurethane von der Art eines "Pfropfcopolymers" mit siloxanhaltigen Seitenketten und einem Polyurethanrückgrat erhalten. Eine Anwendung dieser modifizierten Polyurethane in der Haarkosmetik wird nicht beschrieben.

Die EP-A-0 389 386 beschreibt lineare Diorganopolysiloxan-Polyester-Blockcopolymere mit eingebauten Urethaneinheiten, die sich zur kontrollierten Freigabe pharmazeutischer Zubereitungen eignen.

Die EP-A-0 687 459 beschreibt Haarbehandlungsmittel auf Basis einer wässrigen Polymerdispersion, die erhältlich ist durch radikalische Pfropfcopolymerisation eines monoethylenisch ungesättigten Siloxanmakromonomers und wenigstens eines Polyurethan- und/oder Polyharnstoff-Copolymers. Vernetzte Polyurethane aus Polyurethanpräpolymeren mit endständigen Isocyanatgruppen und Polymerisaten mit gegenüber Isocyanatgruppen reaktiven Gruppen sind in diesem Dokument nicht beschrieben.

Die EP-A-0 773 246 beschreibt wasserlösliche oder wasserdispergierbare Pfropfpolymere aus
A) einem in Wasser löslichen oder dispergierbaren Polyurethanpräpolymer mit endständigen Isocyanatgruppen und
B) einem freie Aminogruppen enthaltenden Protein.

Diese eignen sich als Hilfsmittel in der Kosmetik und insbesondere als Haarfestiger mit verbesserter Auswaschbarkeit. Nachteilig am Einsatz der Proteine ist, dass diese eine Stabilisierung durch Konservierungsmittel erfordern und als Naturstoffe häufig schwankende Produkteigenschaften aufweisen.

Der vorliegenden Erfindung liegt die Aufgabe zugrunde, neue kosmetische Mittel, insbesondere Haarbehandlungsmittel auf Polyurethanbasis, zur Verfügung zu stellen, die einerseits als Haarfestiger brauchbar sind, andererseits aber auch eine gute Auswaschbarkeit (Redispergierbarkeit) besitzen. Vorzugsweise sollen sie dem Haar Glätte und Geschmeidigkeit verleihen.

Überraschenderweise wurde nun gefunden, dass diese Aufgabe durch kosmetische Mittel gelöst wird, die wenigstens ein vernetztes, wasserlösliches oder wasserdispergierbares Polyurethan enthalten, welches das Umsetzungsprodukt aus mindestens einem Polyurethanpräpolymer mit endständigen Isocyanatgruppen und aus mindestens einem Polymerisat mit gegenüber Isocyanatgruppen reaktiven Gruppen darstellt, wobei wenigstens eine der Komponenten eine Siloxangruppe enthält.

Gegenstand der vorliegenden Erfindung ist daher ein kosmetisches Mittel, enthaltend wenigstens ein vernetztes, wasserlösliches oder wasserdispergierbares Polyurethan aus
A) mindestens einem Polyurethanpräpolymer mit endständigen Isocyanatgruppen und
B) mindestens einem Polymerisat, wie in Anspruch 1 definiert, mit gegenüber Isocyanatgruppen reaktiven Gruppen, die ausgewählt sind unter Hydroxyl-, primären und sekundären Amino- und/oder Carboxylgruppen,
wobei mindestens eine der Komponenten A) und/oder B) in Wasser löslich oder dispergierbar ist und wenigstens eine der Komponenten A) und/oder B) mindestens eine Siloxangruppe enthält,
und die Salze davon.

### Polyurethanpräpolymer A)

Bei der Komponente A) handelt es sich bevorzugt um ein Polyurethanpräpolymer aus
a) mindestens einer Verbindung mit einem Molekulargewicht im Bereich von 56 bis 300, die zwei aktive Wasserstoffatome pro Molekül enthält,
b) mindestens einem Polymerisat mit zwei aktiven Wasserstoffatomen pro Molekül,
c) gegebenenfalls einem Polysiloxan,
d) gegebenenfalls mindestens einer Verbindung, die zwei aktive Wasserstoffatome und mindestens eine ionogene bzw. ionische Gruppe pro Molekül aufweist,
e) mindestens einem Diisocyanat.

Bei der Komponente a) handelt es sich bevorzugt um Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein.

Bevorzugt werden als Komponente a) Diole eingesetzt. Brauchbare Diole sind z. B. Ethylenglykol, Propylenglykol, Butylenglykol, Neopentylglykol, Cyclohexandimethylol, Di-, Tri-, Tetra-, Pentaoder Hexaethylenglykol und Mischungen davon. Bevorzugt werden Neopentylglykol und/oder Cyclohexandimethylol eingesetzt.

Geeignete Aminoalkohole sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc.

Geeignete Diamine sind z. B. Ethylendiamin, Propylendiamin, 1,4-Diaminobutan, 1,5-Diaminopentan und 1,6-Diaminohexan sowie α,ω-Diaminopolyether, die durch Aminierung von Polyalkylenoxiden mit Ammoniak herstellbar sind.

Bei der Komponente b) handelt es sich bevorzugt um ein Polymerisat mit einem zahlenmittleren Molekulargewicht im Bereich von 300 bis 5 000, bevorzugt 400 bis 4 000, insbesondere 500 bis 3 000. Brauchbare Polymerisate b) sind z. B. Polyesterdiole, Polyetherole und Mischungen davon. Polyetherole sind vorzugsweise polyalkylenglykole, z. B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Vorzugsweise werden als Komponente b) Polytetrahydrofurane, Polyesterdiole und Mischungen davon eingesetzt.

Geeignete Polytetrahydrofurane b) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Brauchbare Polyesterdiole b) weisen vorzugsweise ein zahlenmittleres Molekulargewicht im Bereich von 400 bis 5 000, bevorzugt 500 bis 3 000, insbesondere 600 bis 2 000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol; Diethylenglykol, Polyethylenglykole, Polypropylenglykole, 1,4-Dimethylolcyclohexan, sowie Poly(meth)acrylatdiole der Formel 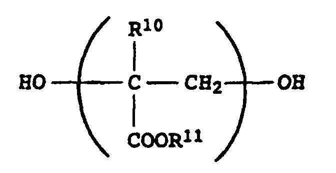 worin R¹⁰ für H oder CH₃ steht und R¹¹ für C₁-C₁₈-Alkyl (insbesondere C₁-C₁₂- oder C₁-C₈-Alkyl) steht, die eine Molmasse von bis zu etwa 3000 aufweisen. Derartige Diole sind auf übliche Weise herstellbar und im Handel erhältlich (Tegomer®-Typen MD, BD und OD der Fa. Goldschmidt).

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% und bevorzugt 50 bis 85 Mol-%, des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/1,6-Hexandiol, 5-NasO₃-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/Dimethylolcyclohexan.

Bei den Polysiloxanen c) handelt es sich vorzugsweise um eine Verbindung der Formel I 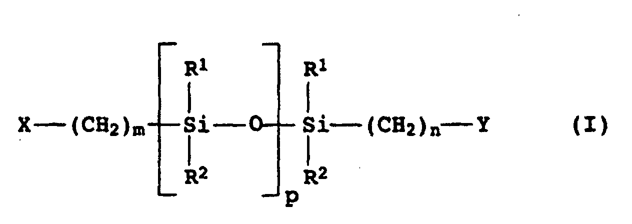 worin
- R¹ und R²: unabhängig voneinander für C₁- bis C₄-Alkyl, Benzyl oder Phenyl stehen,
- X und Y: unabhängig voneinander für OH oder NHR³ stehen, wobei R³ für Wasserstoff, C₁- bis C₆-Alkyl oder C₅- bis C₈-Cycloalkyl steht,
- m und n: unabhängig voneinander für 2 bis 8 stehen,
- p: für 3 bis 50 steht.

Geeignete Alkylreste sind z. B. Methyl, Ethyl, n-Propyl, Isopropyl, n-Butyl, t.-Butyl, n-Pentyl, n-Hexyl etc. Geeignete Cycloalkylreste sind z. B. Cyclopentyl, Cyclohexyl, Cycloheptyl, Cyclooctyl etc.

Vorzugsweise stehen R¹ und R² beide für Methyl.

Diese Polysiloxane c) weisen vorzugsweise ein zahlenmittleres Molekulargewicht im Bereich von 300 bis 5 000, bevorzugt 400 bis 3 000, auf.

Geeignete Verbindungen c) sind auch die in der EP-A-227 816 beschreibenen Polydimethylsiloxane, auf die hiermit Bezug genommen wird.

Geeignete Verbindungen d), die zwei aktive Wasserstoffatome und mindestens eine ionogene oder ionische Gruppe pro Molekül aufweisen, sind z. B. Verbindungen mit Carboxylat- und/oder Sulfonatgruppen. Als Komponente d) sind Dimethylolpropansäure und Mischungen, die Dimethylolpropansäure enthalten, besonders bevorzugt.

Geeignete Diamine und/oder Diole d) mit ionogenen oder ionischen Gruppen sind z. B. Dimethylolpropansäure und Verbindungen der Formel 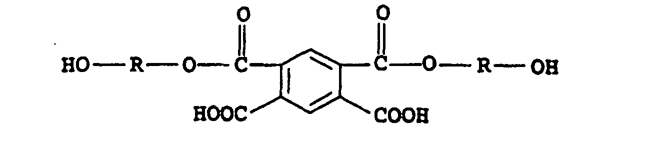 und/oder 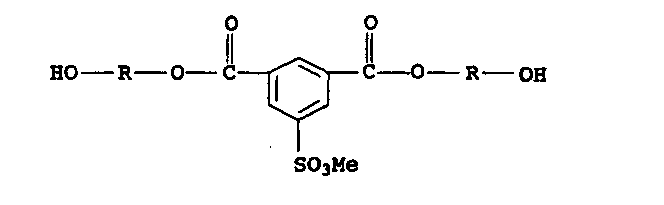 worin R jeweils für eine C₂-C₁₈-Alkylengruppe steht und Me für Na oder K steht.

Als Komponente d) brauchbar sind auch Verbindungen der Formel

H₂N(CH₂)ₙ-NH-(CH₂)ₘ-COO-M⁺

H₂N(CH₂)ₙ-NH-(CH₂)ₘ-SO₃⁻ M⁺

worin m und n unabhängig voneinander für eine ganze Zahl von 1 bis 8, insbesondere 1 bis 6, stehen und M für Li, Na oder K steht, und Verbindungen der Formel

H₂N(CH₂CH₂O)ₚ(CH₂CH(CH₃)O)_{q}(CH₂)ₙ-NH-(CH₂)ₘ-SO₃⁻M⁺

worin m, n und M die zuvor angegebenen Bedeutungen besitzen, p und q unabhängig voneinander für eine ganze Zahl von 0 bis 50 stehen, wobei wenigstens eine der beiden Variablen p oder q > 0 ist. Die Reihenfolge der Alkylenoxideinheiten ist dabei beliebig. Die zuletzt genannten Verbindungen weisen vorzugsweise ein zahlenmittleres Molekulargewicht im Bereich von 400 bis 3 000 auf.

Wenn man als Komponente d) Verbindungen mit stickstoffhaltigen Gruppen einsetzt, erhält man kationische Polyurethane. Brauchbare Komponenten d) sind z. B. Verbindungen der allgemeinen Formeln 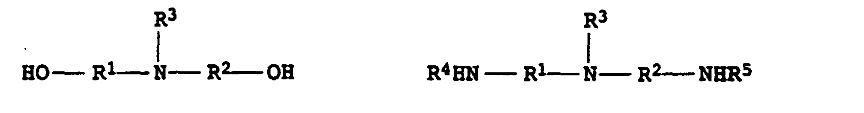 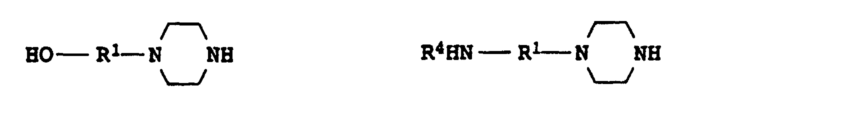 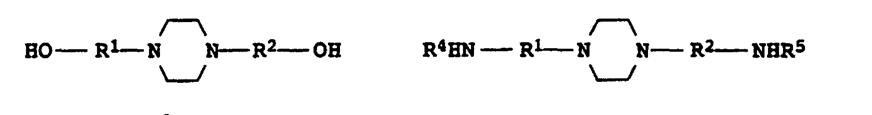 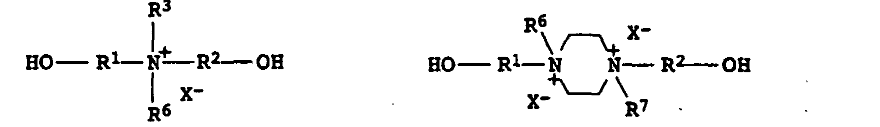 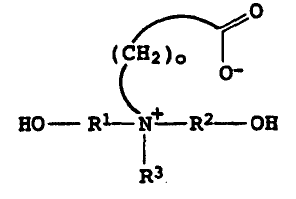 worin
- R¹ und R², die gleich oder verschieden sein können, für C₂-C₈-Alkylen stehen,
- R³, R⁶ und R⁷, die gleich oder verschieden sein können, für C₁-C₆-Alkyl, Phenyl oder Phenyl-C₁-C₄-alkyl stehen,
   R⁴ und R⁵, die gleich oder verschieden sein können, für H oder C₁-C₆-Alkyl stehen,
   o für 1, 2 oder 3 steht,
   X^{⊖} für Chlorid, Bromid, Jodid, C₁-C₆-Alkylsulfat oder SO₄²⁻/₂ steht. Besonders bevorzugt sind N-(C₁- bis C₆-alkyl)diethanolamine, wie Methyldiethanolamin.

Als Komponente d) eignen sich auch Gemische, die mindestens eine der zuvor genannten anionischen oder anionogenen Komponenten und mindestens eine der zuvor genannten kationischen oder kationogenen Komponenten enthalten. Bevorzugt werden dann Gemische eingesetzt, die Dimethylolpropansäure und N-Methyldiethanolamin enthalten.

Bei der Komponente e) handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon, insbesondere Isophorondiisocyanat und/oder Dicyclohexylmethandiisocyanat. Gewünschtenfalls können bis zu 3 Mol-% der genannten Verbindungen durch Triisocyanate ersetzt sein.

Die Herstellung der Polyurethanpräpolymere A) erfolgt durch Umsetzung der Verbindungen der Komponenten a), b) und gegebenenfalls c) und/oder d) mit der Komponente e). Die Temperatur liegt dabei in einem Bereich von etwa 60 bis 140 °C, bevorzugt etwa 70 bis 100 °C. Die Reaktion kann ohne Lösungsmittel oder in einem geeigneten inerten Lösungsmittel oder Lösungsmittelgemisch erfolgen. Geeignete Lösungsmittel sind aprotisch polare Lösungsmittel, z. B. Tetrahydrofuran, Essigsäureethylester, N-Methylpyrrolidon, Dimethylformamid und bevorzugt Ketone, wie Aceton und Methylethylketon. Vorzugsweise erfolgt die Reaktion unter einer Inertgasatmosphäre, wie z. B. unter Stickstoff. Die Komponenten werden bevorzugt in solchen Mengen eingesetzt, dass das Verhältnis von NCO-Äquivalent der Verbindungen der Komponente e) zu Äquivalent aktives Wasserstoffatom der Komponenten a), b) und, falls vorhanden, c) und d) in einem Bereich von etwa 1,0:1 bis 1,4:1, bevorzugt 1,03:1 bis 1,3:1, insbesondere 1,05:1 bis 1,25:1, liegt. Die resultierenden Polyurethanpräpolymere A) weisen somit noch freie Isocyanatgruppen auf.

Vorzugsweise enthalten die Polyurethanpräpolymere A)
- 0,3 bis 15 Gew.-%, bevorzugt 0,5 bis 12 Gew.-%, wenigstens einer Komponente a),
- 0,5 bis 80 Gew.-%, bevorzugt 1 bis 65 Gew.-%, wenigstens einer Komponente b),
- 0 bis 30 Gew.-% wenigstens einer Komponente c),
- 0 bis 25 Gew.-% wenigstens einer Komponente d),
- 25 bis 60 Gew.-%, bevorzugt 35 bis 53 Gew.-%, wenigstens einer Komponente e)
einpolymerisiert.

Polysiloxanhaltige Polyurethanpräpolymere enthalten bevorzugt 0,1 bis 25 Gew.-%, insbesondere 0,2 bis 20 Gew.-%, speziell 0,3 bis 15 Gew.-%, wenigstens einer Komponente c), bezogen auf die Gesamtmenge der Komponenten a) bis e), einpolymerisiert.

Enthalten die Polyurethanpräpolymere wenigstens eine Verbindung d) mit mindestens einer ionogenen bzw. ionischen Gruppe pro Molekül, so ist diese bevorzugt in einer Menge von 5 bis 25 Gew.-%, insbesondere bevorzugt 8 bis 20 Gew.-%, bezogen auf die Gesamtmenge der Komponenten a) bis e), einpolymerisiert.

### Polymerisat B)

Die Herstellung der in den erfindungsgemäßen Mitteln eingesetzten vernetzten, wasserlöslichen oder wasserdispergierbaren Polyurethane erfolgt durch Umsetzung mindestens eines Polyurethanpräpolymers A), wie zuvor beschrieben, mit mindestens einem Polymerisat B).

Das Polymerisat B) wird ausgewählt unter
B1) Polymerisaten, die wenigstens ein α,β-ethylenisch ungesättigtes Monomer einpolymerisiert enthalten, das zusätzlich wenigstens eine gegenüber Isocyanatgruppen reaktive Gruppe pro Molekül enthält,
B2) Polyestern,
B3) Silicon-poly(alkylenoxid)-Copolymeren,
und Mischungen davon.

Bevorzugte Polymerisate B1) enthalten:
f) wenigstens ein α,β-ethylenisch ungesättigtes Monomer, das zusätzlich wenigstens eine gegenüber Isocyanatgruppen reaktive Gruppe pro Molekül enthält,
g) gegebenenfalls wenigstens ein α,β-ethylenisch ungesättigtes Monomer, das ausgewählt ist unter Estern α,β-ethylenisch ungesättigter Mono- und/oder Dicarbonsäuren mit C₁- bis C₂₂-Alkanolen, Amiden α,β-ethylenisch ungesättigter Mono- und/oder Dicarbonsäuren mit Mono- und Di-C₁- bis C₂₂-alkylaminen, Estern von Vinylalkohol und Allylalkohol mit C₁- bis C₄₀-Monocarbonsäuren, Vinylethern, Vinylaromaten, Vinylhalogeniden, Vinylidenhalogeniden C₂- bis C₈-Monoolefinen, nichtaromatischen Kohlenwasserstoffen mit mindestens 2 konjugierten Doppelbindungen und Mischungen davon,
h) gegebenenfalls wenigstens ein α,β-ethylenisch ungesättigtes Monomer, das ausgewählt ist unter N-Vinylamiden, N-Vinyllactamen, primären Amiden α,β-ethylenisch ungesättigter Monocarbonsäuren, vinyl- und allylsubstituierten heteroaromatischen Verbindungen und Mischungen davon,
i) gegebenenfalls wenigstens ein weiteres Monomer mit einer radikalisch polymerisierbaren, α,β-ethylenisch ungesättigten Doppelbindung und mindestens einer ionogenen bzw. ionischen Gruppe pro Molekül
einpolymerisiert.

Geeignete Monomere f) sind die Ester α,β-ethylenisch ungesättigter Mono- und Dicarbonsäuren, wie Acrylsäure, Methacrylsäure, Fumarsäure, Maleinsäure, Itaconsäure, Crotonsäure etc., mit C₁-C₂₀-Alkandiolen. Dazu zählen z. B. 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat etc. Vorzugsweise werden Hydroxyethylacrylat und Hydroxyethylmethacrylat eingesetzt. Geeignete Monomere f) sind auch die Ester der zuvor genannten Säuren mit Triolen und Polyolen, wie z. B. Glycerin, Erythrit, Pentaerythrit, Sorbit etc.

Geeignete Monomere f) sind weiterhin die Ester und Amide der zuvor genannten α,β-ethylenisch ungesättigten Mono- und Dicarbonsäuren mit C₂- bis C₁₂-Aminoalkoholen, die eine primäre oder sekundäre Aminogruppe auf weisen. Dazu zählen Aminoalkylacrylate und Aminoalkylmethacrylate und deren N-Monoalkylderivate, die z. B. einen N-C₁- bis C₈-Monoalkylrest tragen, wie Aminomethylacrylat., Aminomethylmethacrylat, Aminoethylacrylat, Aminoethylmethacrylat, N-Methylaminomethylacrylat, N-Methylaminomethylmethacrylat, N-Ethylaminomethylacrylat, N-Ethylaminomethylmethacrylat, N-(n-propyl)aminomethyl(meth) acrylat, N-Isopropylaminomethyl-(meth)acrylat und bevorzugt tert.-Butylaminoethylacrylat und tert.-Butylaminoethylmethacrylat. Dazu zählen weiterhin N-(Hydroxy-C₁- bis C₁₂-alkyl)(meth)acrylamide, wie N-Hydroxymethyl(meth)-acrylamid, N-Hydroxyethyl(meth)acrylamid etc.

Geeignete Monomere f) sind auch die Amide der zuvor genannten α,β-ethylenisch ungesättigten Mono- und Dicarbonsäuren mit Di- und Polyaminen, die mindestens zwei primäre oder zwei sekundäre oder eine primäre und eine sekundäre Aminogruppe(n) aufweisen. Dazu zählen z. B. die entsprechenden Amide der Acrylsäure und Methacrylsäure (im Folgenden durch die Silbe "(meth)" bezeichnet), wie Aminomethyl(meth)acrylamid, Aminoethyl(meth)acrylamid, Aminopropyl(meth)acrylamid, Amino-n-butyl(meth)acrylamid, Methylaminoethyl(meth)acrylamid, Ethylaminoethyl(meth)acrylamid, Methylaminopropyl(meth)acrylamid, Ethylaminopropyl(meth)acrylamid, Methylamino-n-butyl(meth)acrylamid etc.

Geeignete Monomere g) sind im Wesentlichen hydrophobe, nichtionische Monomere. Dazu zählen die Ester α,β-ethylenisch ungesättigter Mono- und Dicarbonsäuren mit C₁-C₂₂-Alkanolen, bevorzugt C₁-C₁₈-Alkanolen, z. B. die Ester der Acrylsäure und/oder Methacrylsäure mit Methanol, Ethanol, n-Propanol, Isopropanol, n-Butanol, sec.-Butanol, tert.-Butanol, n-Pentanol, n-Hexanol, n-Heptanol, n-Octanol, 2-Ethylhexanol, Dodecanol, Hexadecanol, Octadecanol etc.

Geeignete Monomere g) sind weiterhin Amide α,β-ethylenisch ungesättigter Mono- und Dicarbonsäuren mit Mono- und Dialkylaminen, die 1 bis 22 Kohlenstoffatome, bevorzugt 1 bis 18 Kohlenstoffatome, pro Alkylrest aufweisen. Dazu zählen z. B. N-C₁- bis C₂₂-Alkyl(meth)acrylamide, wie N-Methyl(meth)acrylamid, N-Ethyl(meth)-acrylamid, N-(n-Propyl)(meth)acrylamid, N-Isopropyl(meth)acrylamid, N-Butyl(meth)acrylamid, N-(t.-Butyl)(meth)acrylamid, N-Pentyl(meth)acrylamid, N-Hexyl(meth)acrylamid, N-Heptyl(meth)- acrylamid, N-Octyl(meth) acrylamid, N-Ethylhexyl (meth) acrylamid, N,N-Dimethyl(meth)acrylamid, N,N-Diethyl(meth)acrylamid etc

Geeignete Monomere g) sind weiterhin Vinylformiat, Vinylacetat, Vinylpropionat, Vinyl-n-butyrat, Vinylstearat, Vinyllaurat, Styrol, α-Methylstyrol, o-Chlorstyrol, Vinyltoluole, Vinylchlorid, Vinylidenchlorid, Ethylen, Propylen, Butadien, Isopren, Chloropren, Methyl-, Ethyl-, Butyl-, Dodecylvinylether etc.

Geeignete Monomere h) sind im Wesentlichen hydrophile, nichtionische Monomere. Dazu zählen z. B. N-Vinylamide, wie N-Vinylformamid, N-Vinylacetamid, N-Vinylpropionamid etc. Bevorzugt wird N-Vinylformamid eingesetzt.

Geeignete Monomere h) sind weiterhin N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc.

Geeignete Monomere h) sind weiterhin primäre Amide der zuvor genannten α,β-ethylenisch ungesättigten Monocarbonsäuren, wie Acrylamid, Methacrylamid, Ethacrylamid etc.

Geeignete Monomere h) sind weiterhin vinyl- und allylsubstituierte heteroaromatische Verbindungen, wie 2- und 4-Vinylpyridin, -Allylpyridin, und bevorzugt N-Vinylheteroaromaten, wie N-Vinylimidazol, N-Vinyl-2-methylimidazol etc.

Die Verbindungen i) weisen mindestens eine ionogene bzw. ionische Gruppe pro Molekül auf, die bevorzugt ausgewählt ist unter Carboxylatgruppen und/oder Sulfonatgruppen und deren durch teilweise oder vollständige Neutralisation mit einer Base erhältlichen Salze, sowie tertiäre Amingruppen, die teilweise oder vollständig protoniert und quaternisiert sein können. Geeignete Basen für die Neutralisation bzw. Säuren für die Protonierung und Alkylierungsmittel für die Quaternisierung sind die im Folgenden im Anschluss an die Herstellung der erfindungsgemäßen Polyurethane genannten.

Geeignete Monomere i) sind z. B. die zuvor genannten, α,β-ethylenisch ungesättigten Mono- und/oder Dicarbonsäuren und deren Halbester und Anhydride, wie Acrylsäure, Methacrylsäure, Fumarsäure, Maleinsäure, Itaconsäure, Crotonsäure, Maleinsäureanhydrid, Monobutylmaleat etc. Bevorzugt werden Acrylsäure, Methacrylsäure und deren Alkalimetallsalze, wie deren Natrium- und Kaliumsalze, eingesetzt.

Geeignete Monomere i) sind weiterhin Acrylamidoalkansulfonsäuren und deren Salze, wie 2-Acrylamido-2-methylpropansulfonsäure und deren Alkalimetallsalze, z. B. deren Natrium- und Kaliumsalze.

Weitere geeignete Verbindungen i) sind die Ester der zuvor genannten, α,β-ethylenisch ungesättigten Mono- und Dicarbonsäuren mit C₂- bis C₁₂-Aminoalkoholen, welche am Aminstickstoff C₁- bis c₈-dialkyliert sind. Dazu zählen z. B. N,N-Dimethylaminomethyl-(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat etc. Bevorzugt werden N,N-Dimethylaminopropylacrylat und N,N-Dimethylaminopropyl(meth)acrylat eingesetzt.

Geeignete Monomere i) sind weiterhin die Amide der zuvor genannten α,β-ethylenisch ungesättigten Mono- und Dicarbonsäuren mit Diaminen, die eine tertiäre und ein primäre oder sekundäre Aminogruppe aufweisen. Dazu zählen z. B. N-[2-(dimethylamino)ethyl]-acrylamid, N-[2-(dimethylamino)ethyl]methacrylamid, N-[3-(dimethylamino) propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid etc.

Bevorzugt enthält das Polymerisat B1) zusätzlich zur Komponente f) wenigstens eine Komponente g) und/oder h), sowie gegebenenfalls eine Komponente i) einpolymerisiert.

Bevorzugt enthält das Polymerisat B1)
- 0,05 bis 15 Gew.-%, bevorzugt 0,1 bis 10 Gew.-%, wenigstens einer Komponente f),
- 0 bis 99,9 Gew.-% wenigstens einer Komponente g),
- 0 bis 99,9 Gew.-% wenigstens einer Komponente h),
- 0 bis 50 Gew.-%, bevorzugt 0,1 bis 46 Gew.-%, wenigstens einer Komponente i),
einpolymerisiert.

Vorzugsweise liegt die Gesamtmenge der Komponenten g) und h) in einem Bereich von 30 bis 99,9 Gew.-%, insbesondere 40 bis 99,5 Gew.-%, speziell 50 bis 99,5 Gew.-%.

Nach einer bevorzugten Ausführungsform enthält das Polymerisat B1)
- 0,1 bis 10 Gew.-%, bevorzugt 0,5 bis 5 Gew.-%, wenigstens einer Komponente f)
- 50 bis 99,9 Gew.-%, bevorzugt 60 bis 99,5 Gew.-%, wenigstens einer Komponente h),
- 0 bis 40 Gew.-%, bevorzugt 0 bis 35 Gew.-%, wenigstens einer Komponente i),
einpolymerisiert.

Nach einer weiteren bevorzugten Ausführungsform enthält das Polymerisat B1)
- 0,1 bis 10 Gew.-%, bevorzugt 0,5 bis 5 Gew.-%, wenigstens einer Komponente f),
- 50 bis 99,9 Gew.-%, bevorzugt 60 bis 90 Gew.-%, wenigstens einer Komponente g),
- 0 bis 50 Gew.-%, bevorzugt 10 bis 46 Gew.-%, wenigstens einer Komponente i),_{.}
einpolymerisiert.

Nach einer weiteren bevorzugten Ausführungsform enthält das Polymerisat B1)
- 0,1 bis 10 Gew.-%, bevorzugt 0,5 bis 5 Gew.-%, wenigstens einer Komponente f),
- 0,1 bis 99,9 Gew.-%, bevorzugt. 0,1 bis 90 Gew.-%, wenigstens einer Komponente g),
- 0,1 bis 99,9 Gew.-%, bevorzugt 0,1 bis 99,5 Gew.-%, wenigstens einer Komponente h),
- 0 bis 50 Gew.-%, bevorzugt 0 bis 46 Gew.-%, wenigstens einer Komponente i),
einpolymerisiert.

Die Herstellung der Polymerisate B1) erfolgt nach üblichen, dem Fachmann bekannten Verfahren. Dazu zählt die Polymerisation in Masse und vorzugsweise die Lösungspolymerisation. Die Polymerisationstemperatur beträgt in der Regel 30 bis 120 °C, bevorzugt 40 bis 100 °C. Das Polymerisationsmedium kann sowohl nur aus einem organischen Lösungsmittel als auch aus Mischungen aus Wasser und mindestens einem wassermischbaren, organischen Lösungsmittel bestehen. Bevorzugte organische Lösungsmittel sind z. B. Alkohole, wie Methanol, Ethanol, n-Propanol, Isopropanol, n-Butanol, Ketone, wie Aceton und Methylethylketonen, Tetrahydrofuran etc. Die Lösungspolymerisation kann sowohl als Batchprozess als auch in Form eines Zulaufverfahrens, einschließlich Monomerenzulauf, Stufen- und Gradientenfahrweise, durchgeführt werden. Bevorzugt ist im Allgemeinen das Zulaufverfahren, bei dem man gegebenenfalls einen Teil des Polymerisationsansatzes vorlegt, auf die Polymerisationstemperatur erhitzt und anschließend den Rest des Polymerisationsansatzes, üblicherweise über einen oder auch mehrere, räumliche getrennte Zuläufe, kontinuierlich, stufenweise oder unter Überlagerung eines Konzentrationsgefälles unter Aufrechterhaltung der Polymerisation der Polymerisationszone zuführt.

Als Initiatoren für die radikalische Polymerisation kommen Azoverbindungen in Frage, die für die radikalische Polymerisation geeignet sind. Dazu zählen aliphatische oder cycloaliphatische Azoverbindungen, z. B. 2,2'-Azobis(isobutyronitril), 2,2'-Azobis(2-methylbutyronitril), 2,2'-Azobis(2,4-dimethylvaleronitril), 1,1'-Azobis (1-cyclohexancarbonitril), 2-(Carbamoylazo)isobutyronitril, 4,4'-Azobis(4-cyanovaleriansäure) und deren Alkalimetallund Ammoniumsalze, z. B. das Natriumsalz, Dimethyl-2,2'-azobisisobutyrat, 2,2'-Azobis[2-(2-imidazolin-2-yl)propan], 2,2'-Azobis(2-amidinopropan) und die Säureadditionssalze der beiden zuletzt genannten Verbindungen, z. B. die Dihydrochloride.

Ferner kommen als Initiatoren wasserstoffperoxid, Hydroperoxide in Kombination mit Reduktionsmitteln und Persalze in Frage. Geeignete Hydroperoxide sind beispielsweise t-Butylhydroperoxid, t-Amylhydroperoxid, Cumolhydroperoxid und Pinanhydroperoxid jeweils in Kombination mit beispielsweise einem Salz der Hydroxymethansulfinsäure, einem Eisen (II)-Salz oder Ascorbinsäure. Geeignete Persalze sind insbesondere Alkalimetallperoxidisulfate.

Die verwendete Initiatormenge, bezogen auf die Monomere, liegt im Allgemeinen in einem Bereich von etwa 0,02 bis 15 Mol-%, vorzugsweise 0,05 bis 3 Mol-%.

Sind niedrigere Molekulargewichte erwünscht, so können diese durch Zusatz eines Reglers zum Polymerisationsansatz eingestellt werden. Als Regler eignen sich beispielsweise Aldehyde, wie Formaldehyd, Acetaldehyd, Propionaldehyd, n-Butyraldehyd und Isobutyraldehyd, Ameisensäure, Ammoniumformiat, Hydroxylammoniumsulfat und Hydroxylammoniumphosphat. Weiterhin können Regler eingesetzt werden, die Schwefel in organisch gebundener Form enthalten, wie Di-n-butylsulfid, Di-n-octylsulfid, Diphenylsulfid etc., oder Regler, die Schwefel in Form von SH-Gruppen enthalten, wie n-Butylmercaptan, n-Hexylmercaptan oder n-Dodecylmercaptan. Geeignet sind auch wasserlösliche, schwefelhaltige Polymerisationsregler, wie beispielsweise Hydrogensulfite und Disulfite. Weiterhin eignen sich als Regler Allylverbindungen, wie Allylalkohol oder Allylbromid, Benzylverbindungen, wie Benzylchlorid oder Alkylhalogenide, wie Chloroform oder Tetrachlormethan.

Gewünschtenfalls setzt man der Polymerlösung im Anschluss an die Polymerisationsreaktion einen oder mehrere Polymerisationsinitiatoren zu und erhitzt die Polymerlösung, z. B. auf die Polymerisationstemperatur oder auf Temperaturen oberhalb der Polymerisationstemperatur, um die Polymerisation zu vervollständigen. Geeignet sind die oben angegebenen Azoinitiatoren, aber auch alle anderen üblichen, für eine radikalische Polymerisation in wässriger Lösung geeignete Initiatoren, beispielsweise Peroxide, Hydroperoxide, Peroxodisulfate, Percarbonate, Peroxoester und Wasserstoffperoxid. Hierdurch wird die Polymerisationsreaktion zu einem höheren Umsatz, wie z. B. von 99,9 %, geführt. Die bei der Polymerisation entstehenden Lösungen können gegebenenfalls durch ein dem Stand der Technik entsprechendes Trocknungsverfahren in feste Pulver überführt werden. Bevorzugte Verfahren sind beispielsweise die Sprühtrocknung, die Sprühwirbelschichttrocknung, die Walzentrocknung und die Bandtrocknung. Ebenfalls anwendbar sind die Gefriertrocknung und die Gefrierkonzentrierung. Gewünschtenfalls kann das Lösungsmittel auch durch übliche Methoden, z. B. Destillation bei verringertem Druck, teilweise oder vollständig entfernt und gegebenenfalls gegen das für die folgende Umsetzung des Polymerisats B1) mit dem Polyurethanpräpolymer A) eingesetzte Lösungsmittel ausgetauscht werden. Dabei werden vorzugsweise hydroxylgruppenhaltige Polymerisate B1), die in einem Lösungsmittel mit aktiven Wasserstoffatomen hergestellt wurden, vor der Umsetzung mit A) getrocknet und anschließend in einem Lösungsmittel oder Lösungsmittelgemisch eingesetzt, das keine aktiven Wasserstoffatome aufweist.

Bevorzugt handelt es sich bei der Komponente B2) um einen Polyester auf Basis einer aromatischen Dicarbonsäure oder eines aromatischen Dicarbonsäureanhydrids, wie sie in der DE-A-26 37 167, EP-A-000 171 und insbesondere in der DE-A-42 24 761 beschrieben sind, worauf hiermit in vollem Umfang Bezug genommen wird. Insbesondere handelt es sich bei der Komponente B2) um einen Polyester auf Basis eines Polyesterdiols mit einem zahlenmittleren Molekulargewicht von 500 bis 1 000 und einer aromatischen Di- oder Polycarbonsäure oder eines Carbonsäureanhydrids davon, bevorzugt Trimellithsäureanhydrid. Bevorzugte Diole zur Herstellung der Polyesterdiolkomponente sind die zuvor als Komponenten a) und b) genannten Diole, Polyesterdiole und Polyetherole. Bevorzugte Carbonsäurekomponenten der Polyesterdiole sind aromatische Dicarbonsäuren, wie Phthalsäure, Isophthalsäure oder Terephthalsäure.

Bevorzugt handelt es sich bei der Komponente B3) um eine Verbindung der allgemeinen Formel II 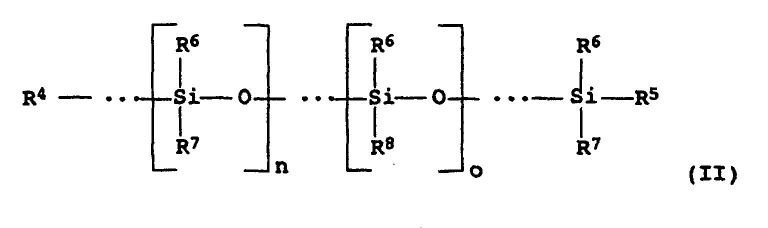 worin
- n und o: unabhängig voneinander für eine ganze Zahl von 0 bis 200 stehen, wobei die Summe aus n und o ≥ 3 ist,
- R⁴, R⁵ und R⁸: unabhängig voneinander für C₁- bis C₈-Alkyl, Benzyl, Phenyl oder einen Rest der Formel III

-(CH₂)ᵣ-O-(CH₂CH₂O)ₚ(CH₂CH(CH₃)O)_{q}-H (III)

stehen,
wobei in der Formel III die Reihenfolge der Alkylenoxideinheiten beliebig ist,
r für eine ganze Zahl von 1 bis 8 steht,
p und q unabhängig voneinander für eine ganze Zahl von 0 bis 200 stehen, wobei die Summe aus p und q > 0 ist,
- R⁶ und R⁷: unabhängig voneinander für C₁- bis C₈-Alkyl, Benzyl oder Phenyl stehen,
wobei die Verbindung der Formel II mindestens zwei Reste der allgemeinen Formel III umfasst.

Vorzugsweise wird die Summe aus n und o so gewählt, dass das Molekulargewicht der Verbindung der Formel II in einem Bereich von etwa 300 bis 30 000 liegt.

Bevorzugt liegt die Gesamtzahl der Alkylenoxideinheiten der Verbindung der Formel II, d. h. die Summe aus p und q in den Formeln III in einem Bereich von etwa 3 bis 200, bevorzugt 5 bis 180.

Bevorzugt sind die Reste R⁶ und R⁷ unabhängig voneinander ausgewählt unter Methyl, Ethyl, n-Propyl, Isopropyl, n-Butyl, sec-Butyl, tert-Butyl, Pentyl, Isopentyl, Hexyl, Octyl, 2-Ethylhexyl, Decyl, Dodecyl und Octadecyl, Cyclohexyl, Phenyl, Naphthyl, Benzyl, Phenylethyl, Tolyl, Xylyl etc.

Bevorzugt steht wenigstens einer der Reste R⁴ oder R⁵, insbesondere bevorzugt beide Reste R⁴ und R⁵, für Methyl.

Geeignete Polymerisate B3) sind Silicon-poly(alkylenoxid)-Copolymere, die unter dem internationalen Freinamen Dimethicon bekannt sind, die Tegopren®-Marken der Fa. Goldschmidt, Belsil® 6031 der Fa. Wacker und Silvet® L der Fa. Witko.

Die Herstellung der erfindungsgemäßen Polyurethane erfolgt durch Umsetzung des Polyurethanpräpolymers A) mit dem Polymerisat B). Dabei liegt das Verhältnis von NCO-Äquivalent der Komponente A) zu Äquivalent aktives Wasserstoffatom der Komponente B) im Allgemeinen in einem Bereich von etwa 20:1 bis 1:1, bevorzugt 10:1 bis 1:1, insbesondere 10:1 bis 1,01:1. Die Temperatur bei der Umsetzung liegt im Allgemeinen in einem Bereich von etwa 10 bis 150 °C, bevorzugt etwa 20 bis 90 °C. Die Reaktion kann vorzugsweise in einem geeigneten inerten Lösungsmittel oder Lösungsmittelgemisch erfolgen. Geeignete Lösungsmittel sind die zuvor für die Herstellung der Polyurethanpräpolymere A) genannten. Wird als Komponente B) ein hydroxylgruppenhaltiges Polymerisat B1) oder B3) oder ein hydroxylgruppen- oder carbonsäuregruppenhaltiger Polyester B2) eingesetzt, so liegt die Reaktionstemperatur bevorzugt in einem Bereich von etwa 60 bis 150 °C. Die Reaktion erfolgt dann vorzugsweise in einem Lösungsmittel oder Lösungsmittelgemisch, welches. keine aktiven Wasserstoffatome aufweist. Bevorzugt werden Ketone, wie Aceton, Methylethylketon und Gemische davon eingesetzt. Wird als Komponente B) ein Polymerisat B1) eingesetzt, das als gegenüber Isocyanatgruppen reaktive Gruppen überwiegend oder ausschließlich primäre und/oder sekundäre Aminogruppen aufweist, so liegt die Reaktionstemperatur bevorzugt in einem Bereich von etwa 20 bis 80 °C. Die Reaktion kann dann gewünschtenfalls in einem Lösungsmittel oder Lösungsmittelgemisch erfolgen, welches aktive Wasserstoffatome aufweisen kann. Neben den zuvor Genannten werden dann bevorzugt Alkohole, wie Methanol und Ethanol, Gemische aus Alkoholen und Wasser sowie Gemische aus Alkoholen und den zuvor. genannten Ketonen eingesetzt. Vorzugsweise wird zur Herstellung der erfindungsgemäßen Polyurethane eine Lösung einer der Komponenten A) oder B) in einem üblichen, dem Fachmann bekannten Reaktor, z. B. einem Rührreaktor, vorgelegt. Die zweite Komponente wird dann vorzugsweise ebenfalls in Form einer Lösung zugegeben und die Umsetzung nach Beendigung der Zugabe solange fortgeführt, bis der NCO-Gehalt des Gemisches konstant bleibt. Weisen die resultierenden Polyurethane noch freie Isocyanatgruppen auf, so werden diese abschließend durch Zusatz von Aminen, vorzugsweise Aminoalkoholen, inaktiviert. Geeignete Aminoalkohole sind die zuvor beschriebenen, bevorzugt 2-Amino-2-methyl-1-propanol.

Die Säuregruppen enthaltenden Polyurethane können mit einer Base teilweise oder vollständig neutralisiert werden. Die Amingruppen enthaltenden Polyurethane können teilweise oder vollständig protoniert oder quaternisiert werden.

In aller Regel weisen die erhaltenen Salze der Polyurethane eine bessere Wasserlöslichkeit oder Dispergierbarkeit in Wasser auf als die nicht neutralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin, C₁-C₆-Alkyldiethyanolamine, bevorzugt Methyl- oder Ethyldiethanolamin und Di-C₁-C₆-Alkylethanolamine. Besonders für den Einsatz in Haarbehandlungsmitteln haben sich zur Neutralisation der Säuregruppen enthaltenden Polyurethane 2-Amino-2-methyl-1-propanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z. B. Mischungen aus Natronlauge und Triisopropanolamin. Die Neutralisation kann je nach Anwendungszweck partiell z. B. zu 20 bis 40 % oder vollständig, d. h. zu 100 % erfolgen.

Die Amingruppen bzw. protonierte oder quaternisierte Amingruppen enthaltenden Polyurethane sind aufgrund ihrer kationischen Gruppen im Allgemeinen leicht in Wasser oder Wasser/Alkohol-Gemischen löslich oder zumindest ohne Zuhilfenahme von Emulgatoren dispergierbar. Geladene kationische Gruppen lassen sich aus den vorliegenden tertiären Aminstickstoffen entweder durch Protonierung, z. B. mit Carbonsäuren, wie Milchsäure, oder Mineralsäuren, wie Phosphorsäure, Schwefelsäure und Salzsäure, oder durch Quaternisierung, z. B. mit Alkylierungsmitteln, wie C₁- bis C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Nach einer geeigneten Ausführungsform können die Polyurethane sowohl Säuregruppen als auch Aminogruppen aufweisen. Der Betrag der Differenz aus Säuregruppen und Aminogruppen (|ΔSZ-AZ|) liegt dabei vorzugsweise in einem Bereich von etwa 15 bis 150, bevorzugt 30 bis 100. Säurezahl und Aminzahl sind dabei jeweils als mg KOH/g Prüfsubstanz definiert.

Wird bei der Herstellung der Polyurethane ein wassermischbares organisches Lösungsmittel eingesetzt, so kann dieses im Anschluss durch übliche, dem Fachmann bekannte Verfahren, z. B. durch Destillation bei vermindertem Druck, entfernt werden. Vor dem Abtrennen des Lösungsmittels kann dem Polyurethan zusätzlich Wasser zugegeben werden. Nach Ersatz des Lösungsmittels durch Wasser erhält man eine Lösung oder Dispersion des Polymers, aus der, falls gewünschte das Polymer in üblicher Weise gewonnen werden kann, z. B. durch Sprühtrocknung.

Die in den erfindungsgemäßen Mitteln eingesetzten Polyurethane weisen einen auf das Gesamtgewicht der eingebauten Komponenten bezogenen Siloxangehalt, entsprechend dem Gewichtsanteil der eingebauten Verbindungen der allgemeinen Formeln I und/oder II, von 0,05 bis 30 Gew.-%,bevorzugt 0,05 bis 25 Gew.-%, insbesondere 0,05 bis 20 Gew.-%, speziell 0,1 bis 20 Gew.-%, auf. Ihre K-Werte (gemessen nach E. Fikentscher, Cellulose-Chemie 13 (1932), S. 58-64, an einer 1%igen Lösung in N-Methylpyrrolidon) liegen im Allgemeinen in einem Bereich von 15 bis 90, bevorzugt 20 bis 60. Ihre Glasübergangstemperatur beträgt im Allgemeinen mindestens 0 °C, bevorzugt mindestens 20 °C, insbesondere bevorzugt mindestens 25 °C und speziell mindestens 30 °C. Weisen die erfindungsgemäßen Polyurethane zwei oder mehrere Glasübergangstemperaturen auf, so liegt wenigstens eine davon in dem angegebenen Bereich. Bevorzugt liegt/liegen die andere(n) dann unterhalb des zuvor angegebenen Temperaturbereichs.

Mittel, enthaltend wenigstens ein Polyurethan mit einem Siloxangehalt im Bereich von 5 bis 25 Gew.-%, bevorzugt 7 bis 20 Gew.-%, werden vorzugsweise als Lösungsvermittler für hydrophobe Produkte, insbesondere Silicone, und als Additive für Haarbehandlungsmittel eingesetzt. Mittel, enthaltend wenigstens ein Polyurethan mit einem Siloxangehalt im Bereich von etwa 0,05 bis 15 Gew.-% werden bevorzugt in Form eines Haarbehandlungsmittels, insbesondere in Form eines Haarsprays, eingesetzt.

Die in den erfindungsgemäßen Mitteln enthaltenen Polyurethane sind als Hilfsmittel in der Kosmetik und Pharmazie, insbesondere als oder in Beschichtungsmittel(n) für keratinhaltige Oberflächen (Haar, Haut und Nägel) und als Überzugsmittel und/oder Bindemittel für feste Arzneiformen brauchbar. Außerdem sind sie als oder in Beschichtungsmittel(n) für die Textil-, Papier-, Druck-, Leder- und Klebstoffindustrie brauchbar. Sie sind insbesondere in der Haarkosmetik brauchbar. Die zuvor genannten Polyurethane können auch in Cremes und als Tablettenüberzugmittel und Tablettenbindemittel verwendet werden. Sie eignen sich auch als Bindemittel und Klebemittel für kosmetische Produkte, z. B. bei der Herstellung stiftförmiger, kosmetischer Produkte, wie Deostifte, Schminkstifte etc.

Die erfindungsgemäßen kosmetischen Mittel eignen sich insbesondere als Beschichtungsmittel für keratinhaltige Oberflächen (Haar, Haut und Nägel). Die in ihnen eingesetzten Verbindungen sind wasserlöslich oder wasserdispergierbar. Sind die in den erfindungsgemäßen Mitteln eingesetzten Verbindungen wasserdispergierbar, können sie in Form von wässrigen Mikrodispersionen mit Teilchendurchmessern von üblicherweise 1 bis 250 nm, bevorzugt 1 bis 150 nm, zur Anwendung gebracht werden. Die Feststoffgehalte der Präparate liegen dabei üblicherweise in einem Bereich von etwa 0,5 bis 20 Gew.-%, bevorzugt 1 bis 12 Gew.-%. Diese Mikrodispersionen benötigen in der Regel keine Emulgatoren oder Tenside zu ihrer Stabilisierung.

Bevorzugt können die erfindungsgemäßen Mittel in Form eines Haarbehandlungsmittels, insbesondere in Form eines Haarsprays vorliegen. Zur Anwendung als Haarfestiger sind dabei Mittel bevorzugt, die Polyurethane enthalten, die wenigstens eine Glasübergangstemperatur T_{g} ≥ 20 °C, bevorzugt ≥ 30 °C, aufweisen. Der K-Wert dieser Polymere liegt vorzugsweise in einem Bereich von 23 bis 90, insbesondere 25 bis 60.

Im Allgemeinen enthalten die erfindunsgemäßen Mittel die Polyurethane in einer Menge im Bereich von 0,2 bis 20 Gew.-%, bezogen auf das Gesamtgewicht des Mittels.

Vorzugsweise handelt es sich um Haarbehandlungsmittel. Diese liegen üblicherweise in Form einer wässrigen Dispersion oder in Form einer alkoholischen oder wässrig-alkoholischen Lösung vor. Beispiele geeigneter Alkohole sind Ethanol, Propanol, Isopropanol etc.

Weiter enthalten die erfindungsgemäßen Haarbehandlungsmittel im Allgemeinen übliche kosmetische Hilfsstoffe, beispielsweise Weichmacher, wie Glycerin und Glykol; Emollienzien; Parfüms; UV-Absorber; Farbstoffe; antistatische Mittel; Mittel zur Verbesserung der Kämmbarkeit; Konservierungsmittel; und Entschäumer.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu erhöhen. Sie beträgt dann im Allgemeinen nicht mehr als 55 Gew.-%, bezogen auf das Gesamtgewicht des Mittels. Gewünschtenfalls sind aber auch höhere VOC-Gehalte von 85 Gew.-% und darüber möglich.

Die zuvor beschriebenen Polyurethane können auch in Kombination mit anderen Haarpolymeren in den Mitteln zur Anwendung kommen. Solche Polymere sind insbesondere:
- nicht-ionische, wasserlösliche bzw. wasserdispergierbare Polymere oder Oligomere, wie Polyvinylcaprolactam, z. B. Luviskol Plus (BASF), oder Polyvinylpyrrolidon und deren Copolymere, insbesondere mit Vinylestern, wie Vinylacetat, z. B. Luviskol VA 37 (BASF); Polyamide, z. B. auf Basis von Itaconsäure und aliphatischen Diaminen;
- amphotere oder zwitterionische Polymere, wie die unter den Bezeichnungen Amphomer® (Delft National) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind und Copolymere aus Hydroxyethylmethacrylat, Methylmethacrylat, N,N-Dimethylaminoethylmethacrylat und Acrylsäure (Jordapon®);
- anionische Polymere, wie vinylacetat/Crotonsäure-Copolymere, wie sie beispielsweise unter den Bezeichnungen Resyn® (NATIONAL STARCH), Luviset® (BASF) und Gafset® (GAF) im Handel sind, Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer, Acrylsäure/Ethylacrylat/N-tert.Butylacrylamid-Terpolymere, die beispielsweise unter der Bezeichnung Ultrahold® strong (BASF) vertrieben werden, sowie Luvimer® (BASF, Terpolymer aus t-Butylacrylat, Ethylacrylat und Methacrylsäure), oder
- kationische (quaternisierte) Polymere, z. B. kationische Polyacrylatcopolymere auf Basis von N-Vinyllactamen und deren Derivaten (N-Vinylpyrrolidon,.N-Vinylcaprolactam etc.) sowie übliche kationische Haarconditionerpolymere, z. B. Luviquat®) (Copolymer aus Vinylpyrrolidon und Vinylimidazoliummethochlorid), Luviquat®) Hold (Copolymerisat aus quaternisiertem N-Vinylimidazol, N-Vinylpyrrolidon und N-Vinylcaprolactam), Merquat®) (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.;
- nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Die erfindungsgemäßen vernetzten siloxangruppenhaltigen Polyurethane können als Mischung mit einem anderen siloxanfreien amidgruppenhaltigen Haarpolymer eingesetzt werden. Dazu zählen z. B. die in der DE-A-42 25 045 beschriebenen Polyurethane, die zuvor beschriebenen Vinylpyrrolidon/Acrylat-Terpolymere und Acrylsäure/Ethylacrylat/N-tert.-Butylacrylamid-Terpolymere (z. B. Ultrahold®strong der BASF AG), die in der DE-A-42 41 118 beschriebenen kationischen Polyurethane, die zuvor beschriebenen amidgruppenhaltigen amphoteren Polymere (z. B. Amphomer®) und insbesondere Copolymerisate, die einen Anteil an amidgruppenhaltigen Monomeren, wie N-Vinyllactamen, von mindestens 30 Gew.-% aufweisen (z. B. Luviskol®plus und Luviskol®VA37 der BASF AG).

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel enthält:
a) 0,5 bis 20 Gew.-% mindestens eines, in Wasser löslichen oder dispergierbaren, siloxanhaltigen Polyurethans,
b) 40 bis 99 Gew.-%, bevorzugt 50 bis 98 Gew.-%, eines Lösungsmittels, ausgewählt unter Wasser und wassermischbares Lösungsmitteln, bevorzugt C₂- bis C₅-Alkoholen, insbesondere Ethanol, und Mischungen davon,
c) 0 bis 50 Gew.-% eines Treibmittels, vorzugsweise Dimethylether,
d) 0 bis 15 Gew.-% mindestens eines von a) verschiedenen, in Wasser löslichen oder dispergierbaren Haarpolymers,
e) 0 bis 0,2 Gew.-% mindestens eines wasserunlöslichen Silicons,
f) 0 bis 2 Gew.-% mindestens eines nichtionischen, siloxanhaltigen, in Wasser löslichen oder dispergierbaren Polymers,
sowie übliche Zusatzstoffe.

Das erfindungsgemäße Mittel kann als Komponente d) mindestens ein anderes, in Wasser lösliches oder dispergierbares Haarpolymer enthalten. Der Anteil dieser Komponente beträgt dann im Allgemeinen etwa 0,1 bis 15 Gew.-%, bevorzugt 0,1 bis 10 Gew.-%, bezogen auf das Gesamtgewicht des Mittels. Bevorzugt können dabei wasserlösliche oder wasserdispergierbare Polyurethane eingesetzt werden, die keine Siloxangruppen einpolymerisiert enthalten.

Das erfindungsgemäße Mittel kann als Komponente e) mindestens ein wasserunlösliches Silicon, insbesondere ein Polydimethylsiloxan, z. B. die Abil®-Typen der Fa. Goldschmidt, enthalten. Der Anteil dieser Komponente beträgt dann im Allgemeinen etwa 0,0001 bis 0,2 Gew.-%, bevorzugt 0,001 bis 0,1 Gew.-%, bezogen auf das Gesamtgewicht des Mittels.

Das erfindungsgemäße Mittel kann als Komponente f) mindestens ein nichtionisches, siloxanhaltiges, wasserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Das erfindungsgemäße Mittel kann zusätzlich gegebenenfalls einen Entschäumer, z. B. auf Silicon-Basis, enthalten. Die Menge des Entschäumers beträgt im Allgemeinen bis zu etwa 0,001 Gew.-%, bezogen auf die Gesamtmenge des Mittels.

Die erfindungsgemäßen Mittel besitzen den Vorteil, dass sie einerseits den Haaren die gewünschte Festigkeit verleihen und andererseits die Polymere leicht auswaschbar (redispergierbar) sind. Darüber hinaus lassen sich Haarbehandlungsmittel mit einem in VOC-Gehalt von weniger als 85 Gew.-%, bevorzugt weniger als 60 Gew.-%, und auch rein wässrige Formulierungen herstellen, selbst wenn sie als Haarspray formuliert sind.

Die Erfindung wird anhand der folgenden, nicht einschränkenden Beispiele näher erläutert.

### Beispiele

### Beispiele 1 bis 4

### Polyurethanpräpolymerherstellung

In einem Rührapparat, der mit Rührer, Tropftrichter, Thermometer, Rückflusskühler und einer Vorrichtung für das Arbeiten unter Stickstoff ausgestattet war, wurden ein Polyesterdiol (Mₙ = 1 000 g/mol, hergestellt aus Isophthalsäure, Adipinsäure und Hexandiol), Neopentylglykol, gegebenenfalls Dimethylolpropansäure (Beispiele 1, 2 und 4) und gegebenenfalls N-Methyldiethanolamin (Beispiel 4) in einer Menge nach Tabelle 1 in Methylethylketon (Feststoffgehalt der resultierenden Reaktionslösung ca. 75 %) unter Erhitzen auf eine Temperatur von etwa 70 °C und unter Rühren gelöst. Anschließend wurde unter Rühren Isophorondiisocyanat in einer Menge nach Tabelle 1 zugetropft, wobei die Reaktionstemperatur anstieg. Bei einer Innentemperatur von 85 °C wurde das Reaktionsgemisch dann solange gerührt, bis der Isocyanatgruppengehalt des Gemisches praktisch konstant blieb (ca. 0,5 bis 1 %) und anschließend unter Rühren auf Raumtemperatur abgekühlt. Gegebenenfalls gab man dann bei einer Temperatur von etwa 30 °C ein Polysiloxandiamin (Mₙ = 900 g/mol, Tegomer® A-Si 2122 der Fa. Goldschmidt, in Form einer 80%igen Lösung in Methylethylketon) (Beispiele 1, 2, 3) in einer Menge nach Tabelle 1 zu dem wie zuvor beschrieben hergestellten Polyurethanpräpolymer. Die Mischung wurde dann noch weitere 30 Minuten umgesetzt. In allen Fällen wurde das Reaktionsgemisch mit Methylethylketon auf 40 Gew.-% verdünnt.

**Tabelle 1:**

| Bsp. Nr. | Polyesterdiol¹⁾ [mol] | Polysiloxandiamin²⁾ [mol] | NPG³⁾ [mol] | DMPA⁴⁾ [mol] | MDEA⁵⁾ [mol] | IPDI⁶⁾[mol] | Siloxangehalt [Gew.-%] |
|---|---|---|---|---|---|---|---|
| 1 | 0,6 | 0,2 | 1,7 | 3 | - | 6 | 6,7 |
| 2 | 0,6 | 0,7 | 1,2 | 3 | - | 6 | 20,4 |
| 3 | 1,0 | 1,0 | 1,0 | - | - | 4 | 31,1 |
| 4 | 1,2 | - | 1,4 | 2,5 | 0,5 | 6 | 0 |

| | | | | | | | |
|---|---|---|---|---|---|---|---|
| 1) Polyesterdiol aus Isophthalsäure, Adipinsäure, Hexandiol, Mₙ = 1 000 g/mol | | | | | | | |
| 2) Polysiloxandiamin, Mₙ = 900 g/mol (Tegomer® A-Si 2122 der Fa. Goldschmidt) | | | | | | | |
| 3) NPG = Neopentylglykol | | | | | | | |
| 4) DMPA = Dimethylolpropansäure | | | | | | | |
| 5) MDEA = N-Methyldiethanolamin | | | | | | | |
| 6) IPDI = Isophorondiisocyanat | | | | | | | |

### Beispiele 5 bis 21

### Herstellung des Polymerisates B1)

| | | |
|---|---|---|
| Zulauf 1 | 300 g | Monomerengemisch nach Tabelle 2 |
| | 30 g | Lösungsmittel: Beispiele 5, 6, 8-13, 17-21: Ethanol Beispiele 7, 14-16: Ethanol/Wasser (1:1) |
| | | |
| Zulauf 2 | 0,6 g | 2,2'-Azobis(2-methylbutyronitril) |
| | 150 g | Ethanol |
| | | |
| Zulauf 3 | 3,0 g | 2,2'-Azobis(2-methylbutyronitril) |
| | 150 g | Ethanol |

In einer Rührapparatur mit Rückflusskühler und zwei separaten Zulaufvorrichtungen wurden 20 Gew.-% von Zulauf 1 (Monomerengemisch gemäß Tabelle 2), 12 Gew.-% von Zulauf 2 und 120 g Ethanol vorgelegt und die Mischung auf ca. 75 °C aufgeheizt. Nach dem Anpolymerisieren, erkennbar an einer beginnenden Viskositätserhöhung, wurde der Rest von Zulauf 1 innerhalb von 4 Stunden und der Rest von Zulauf 2 innerhalb von 5 Stunden zugegeben, wobei die Innentemperatur auf etwa 70 bis 75 **°C** gehalten wurde. Anschließend wurde Zulauf 3 innerhalb von 2 Stunden zugegeben, wobei die Innentemperatur auf ca. 80 °C erhöht wurde. Nach dem Ende der Zugabe wurde noch ca. 5 Stunden bei dieser Temperatur nachpolymerisiert. Die so erhaltenen Polymerisate können ohne weitere Maßnahmen zur Verringerung des Restmonomerengehalts zur Polyurethanherstellung eingesetzt werden. Polymerisate mit sehr geringen Restmonomergehalten werden erhalten, wenn man statt Zulauf 3 z. B. 2,5-Bis-(tert.-butylperoxy-2,5-dimethylhexan) (Trigonox®) 101 der Fa. Akzo Nobel) in 150 g Ethanol innerhalb von 2 Stunden zu dem Reaktionsgemisch gibt und anschließend noch etwa 5 Stunden bei einer Temperatur von ca. 130 °C unter dem Eigendruck des Reaktionsgemisches nachpolymerisiert.

Die hydroxylgruppenhaltigen Polymerisate der Beispiele 5 bis 8 werden im Anschluss an die Polymerisation durch Sprühtrocknung getrocknet und dann werden für die weitere Umsetzung 40 gew.-%ige Lösungen dieser Polymerisate in Methylethylketon hergestellt.

Bei den in einem Lösungsmittelgemisch aus Ethanol/wasser (1:1) hergestellten Polymerisaten der Beispiele 14 bis 16 wird das Lösungsmittel durch Destillation unter verringertem Druck bei etwa 40 °C entfernt und dann werden für die weitere Umsetzung 40 gew.-%ige Lösungen in Ethanol hergestellt.

Die ethanolischen Lösungen der übrigen Polymerisate werden durch Zugabe von weiterem Ethanol ebenfalls auf 40 Gew.-% eingestellt.

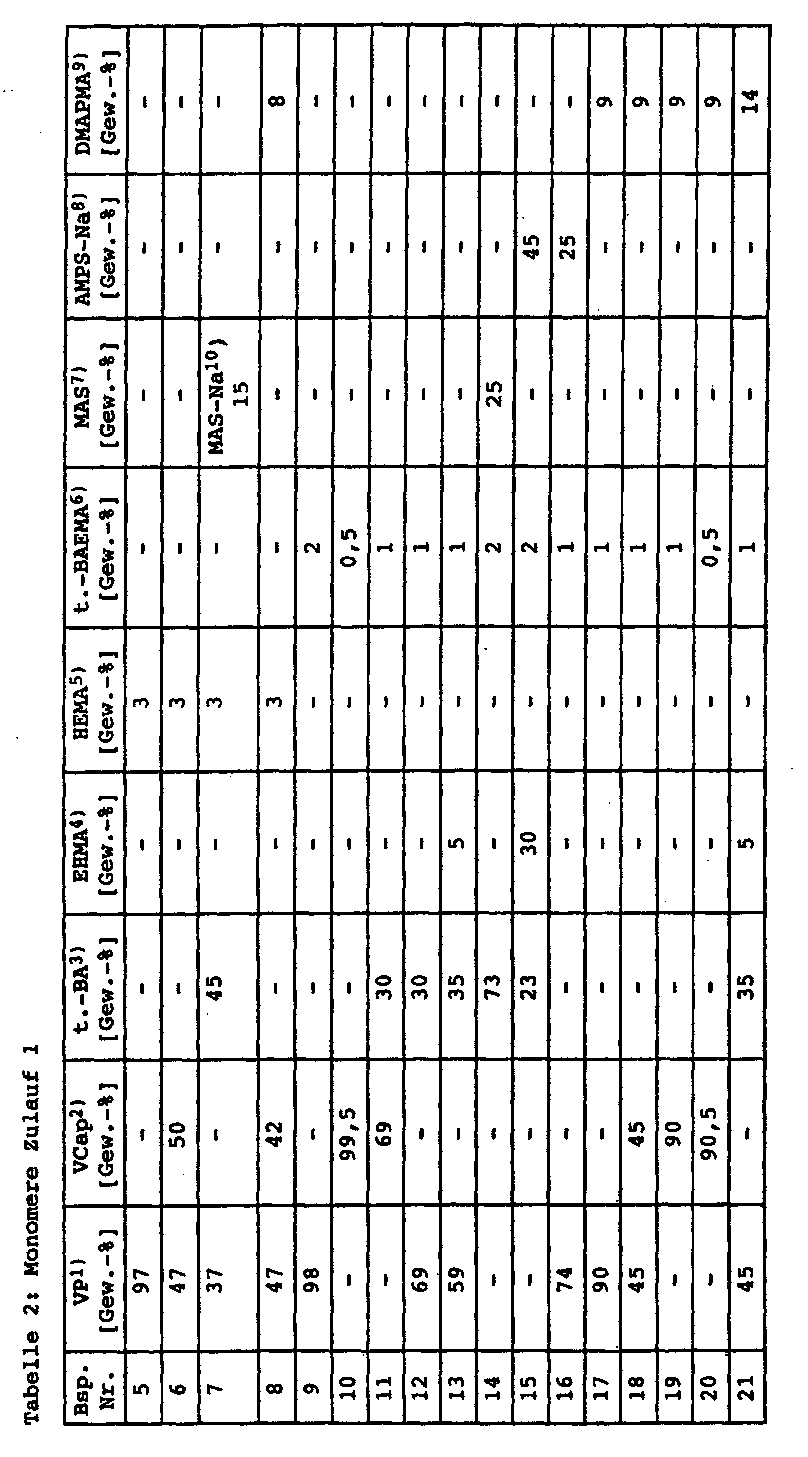

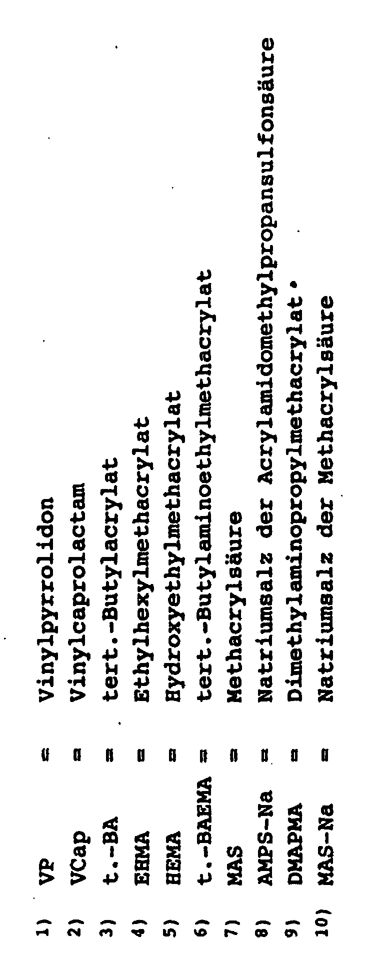

### Beispiele 22 und 23

### Herstellung des Polyesters B2)

In einem Vierhalskolben, der mit Rührer, Innenthermometer, absteigendem Kühler und einer Vorrichtung für das Arbeiten unter Stickstoff versehen war, wurden Diethylenglykol, 1,4-Cyclohexandimethylol, gegebenenfalls Polypropylenglykol (Mₙ = 600 g/mol) (Beispiel 23) und Neopentylglykol in einer Menge nach Tabelle 3 sowie 100 ppm Tetrabutylorthotitanat als Katalysator vorgelegt. Unter leichtem Stickstoffstrom wurde das Reaktionsgemisch unter Erhitzen auf eine Temperatur von etwa 80 °C und unter Rühren gelöst. Dann wurde eine Menge Isophthalsäure nach Tabelle 3 zugegeben und anschließend 2 Stunden auf 160 °C erhitzt. Anschließend wurde die Temperatur mit 20 °C/h erhöht und bei etwa 220 °C das Reaktionswasser abdestilliert, bis. die Säurezahl unter 15 gefallen war. Anschließend gab man Trimellithsäureanhydrid in einer Menge nach Tabelle 3 und 100 ppm weiteren Katalysator zu und ließ noch 4 Stunden bei einer Temperatur von etwa 200 **°C** und einem verminderten Druck von etwa 1.33 kPa [10 mm Hg] weiterreagieren. Nach dem Abkühlen auf etwa 70 °C wurde ein hellgelber Polyester erhalten, der mit Methylethylketon zu einer 70 gew.-%igen Lösung formuliert wurde.

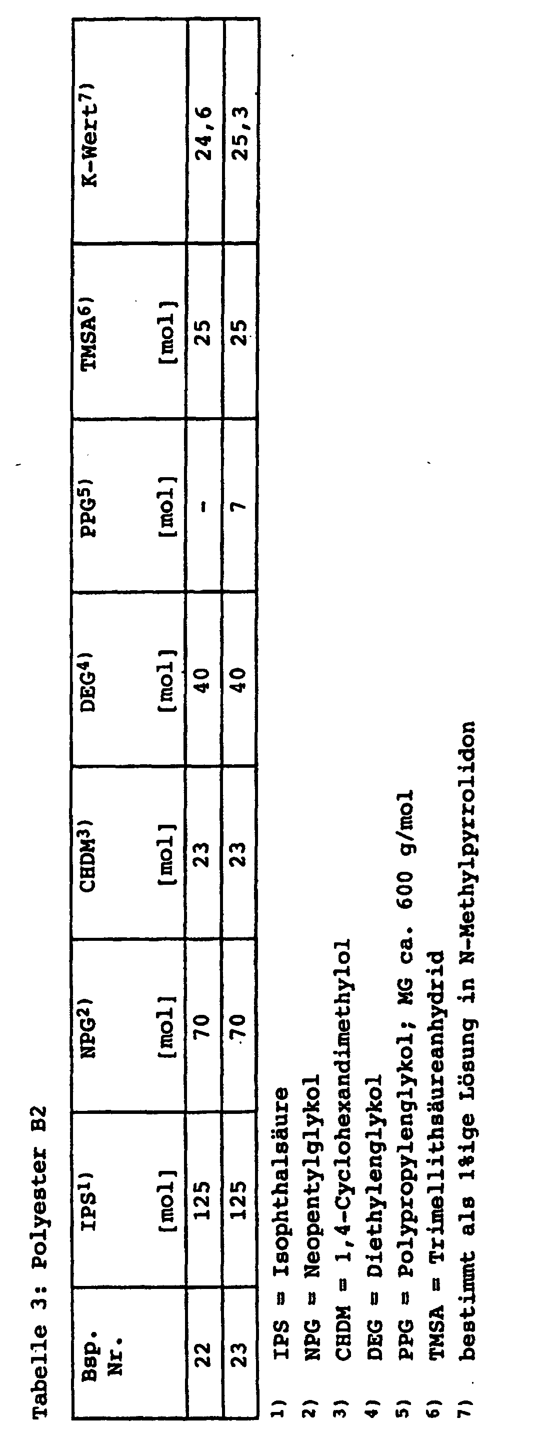

### Beispiele 24 bis 28 und 46 bis 48

### Polyurethanherstellung

In einem Rührapparat, der mit Rührer, Tropftrichter, Thermometer und Rückflusskühler ausgestattet war, wurde eine 40 gew.-%ige Lösung (in Methylethylketon) eines Polyurethanpräpolymers aus Beispiel 1, 2 oder 4, wie in Tabelle 4 angegeben, vorgelegt und auf 60 °C erhitzt. Anschließend wurde bei den Beispielen 24 bis 28 eine 40 gew.-%ige Lösung eines hydroxylgruppenhaltigen Polymerisates B1) aus Beispiel 5 bis 8, ebenfalls in Methylethylketon, wie in Tabelle 4 angegeben, zugemischt. Das Reaktionsgemisch wurde bei einer Temperatur von etwa 85 °C solange gerührt, bis der Isocyanatgruppengehalt des Gemisches praktisch konstant blieb (etwa 2 Stunden). Anschließend gab man Wasser zum Reaktionsgemisch und neutralisierte das Reaktionsprodukt mit 2-Amino-2-methylpropanol (pH etwa 8,0). Das Methylethylketon wurde dann im Vakuum bei 40 °C abdestilliert, wobei eine wässrige Dispersion des Polyurethans erhalten wurde.

Bei den Beispielen 46 bis 48 wurde analog verfahren, wobei anstelle des hydroxylgruppenhaltigen Polymerisates B1) ein Siliconpoly(alkylenoxid)-Copolymer B3) (Belsil® 6031 der Fa. wacker) als 40 gew.-%ige Lösung in Methylethylketon eingesetzt wurde.

Pulverförmige Produkte können durch Sprühtrocknung erhalten werden.

### Beispiele 29 bis 43

In einem Rührapparat, der mit Rührer, Tropftrichter, Thermometer und Rückflusskühler ausgestattet war, wurde eine 40 gew.-%ige Lösung eines Polyurethanpräpolymers aus Beispiel 1 bis 3 in Methylethylketon, gemäß Tabelle 4, vorgelegt. Bei einer Temperatur von etwa 30 °C wurde dann ein Polymerisat B1) (oder ein Gemisch, Beispiel 43), gemäß Tabelle 4, in Form einer 40 gew.-%igen Lösung in Ethanol zugemischt. Das Reaktionsgemisch wurde dann etwa 1 Stunde bei Umgebungstemperatur gerührt. Anschließend gab man Wasser zum Reaktionsgemisch und neutralisierte das Reaktionsprodukt mit 2-Amino-2-methylpropanol (pH etwa 8,0). Das Methylethylketon wurde dann im Vakuum bei 40 °C abdestilliert, wobei eine wässrige Dispersion des Polyurethans erhalten wurde.

Ein pulverförmiges Produkt kann durch Sprühtrocknung erhalten werden.

### Beispiele 44 und 45

In einem Rührapparat, der mit Rührer, Tropftrichter, Thermometer und Rückflusskühler ausgestattet war, wurde eine 40 gew.-%ige Lösung eines Polyurethanpräpolymers aus Beispiel 2 oder 3 in Methylethylketon, wie in Tabelle 4 angegeben, vorgelegt und auf 70 °C erhitzt. Anschließend wurde eine 70 gew.-%ige Lösung eines Polyesters B2) (aus Beispielen 22 und 23) in Methylethylketon in einer Menge nach Tabelle 4 zugemischt. Das Reaktionsgemisch wurde bei einer Temperatur von etwa 70 °C so lange gerührt, bis der Isocyanatgruppengehalt = 0 war (etwa 2 Stunden). Anschließend wurden noch enthaltene freie Carbonsäuregruppen mit 2-Amino-2-methylpropanol neutralisiert (pH etwa 8,2). Dann gab man Wasser zum Reaktionsgemisch und destillierte das Methylethylketon unter Zugabe eines Tropfens Silicon-Entschäumers bei 40 °C ab, wobei eine stabile wässrige Dispersion erhalten wurde.

Ein pulverförmiges Produkt kann durch Sprühtrocknung erhalten werden.

**Tabelle 4 :**

| Bsp. Nr. | PU-Präpolymer | | Polymerisat B) | | K-Wert¹⁾ |
|---|---|---|---|---|---|
| | Bsp. Nr. | [Gew.-%] | Bsp. Nr. | [Gew.-%] | |
| 24 | 1 | 80 | 5 | 20 | 33,0 |
| 25 | 1 | 50 | 6 | 50 | 37,3 |
| 26 | 1 | 90 | 7 | 10 | 32,4 |
| 27 | 1 | 90 | 8 | 10 | 34,7 |
| 28 | 2 | 90 | 8 | 10 | 36,1 |
| 29 | 1 | 90 | 9 | 10 | 34,7 |
| 30 | 1 | 70 | 10 | 30 | 35,4 |
| 31 | 1 | 90 | 14 | 10 | 37,6 |
| 32 | 1 | 80 | 16 | 20 | 39,4 |
| 33 | 1 | 80 | 17 | 20 | 31,2 |
| 34 | 1 | 80 | 18 | 20 | 35,3 |
| 35 | 1 | 70 | 20 | 30 | 32,1 |
| 36 | 1 | 80 | 21 | 20 | 37,4 |
| 37 | 2 | 80 | 18 | 20 | 32,0 |
| 38 | 1 | 10 | 21 | 90 | 46,2 |
| 39 | 1 | 20 | 10 | 80 | 42,0 |
| 40 | 1 | 30 | 20 | 70 | 41,0 |
| 41 | 3 | 10 | 14 | 90 | 39,7 |
| 42 | 3 | 10 | 17 | 90 | 43,9 |
| 43 | 3 | 10 | 13 14 | 30 60 | 40,0 |
| 44 | 3 | 6,67 | 22 | 93,33 | 26,2 |
| 45 | 2 | 20 | 23 | 80 | 25,8 |
| 46 | 4 | 95 | Silicon | 5 | 30,2 |
| 47 | 4 | 80 | Silicon | 20 | 25,2 |
| 48 | 2 | 80 | Silicon | 20 | 24,7 |

| | | | | | |
|---|---|---|---|---|---|
| ¹⁾ 1 gew.-%ige Lösung in N-Methylpyrrolidon | | | | | |

### Anwendungstechnische Beispiele

### Beispiele 49 bis 69

Aerosol-Haarspray-Formulierungen mit einem VOC-Gehalt von 97 Gew.-%:

### Polyurethan gemäß Beispielen

| | |
|---|---|
| 24-27, 29-36, 38-46 | 3,00 Gew.-% |
| Ethanol | 62,00 Gew.-% |
| Dimethylether | 34,96 Gew.-% |
| Parfüm, Zusatzstoffe | q.s. |

### Beispiele 70 bis 90

Kompakte Aerosol-Haarspray-Formulierungen mit einem VOC-Gehalt von 90 Gew.-%:

### Polyurethan gemäß Beispielen

| | |
|---|---|
| 24-27, 29-36, 38-46 | 10,00 Gew.-% |
| Ethanol | 55,00 Gew.-% |
| Dimethylether | 34,96 Gew.-% |
| Parfüm, Zusatzstoffe | q.s. |

### Beispiele 91 bis 111

Haarspray-Formulierungen mit einem VOC-Gehalt von 80 Gew.-%:

### Polyurethan gemäß Beispielen

| | |
|---|---|
| 24-27, 29-36, 38-46 | 5,00 Gew.-% |
| Ethanol | 45,00 Gew.-% |
| Wasser | 15,00 Gew.-% |
| Dimethylether | 34,96 Gew.-% |
| Parfüm, Zusatzstoffe | q.s. |

### Beispiele 112 bis 126

Haarspray-Formulierungen mit einem VOC-Gehalt von 55 Gew.-%:

### Polyurethan gemäß Beispielen

| | |
|---|---|
| 24-27, 29-36, 44-46 | 5,00 Gew.-% |
| Ethanol | 20,00 Gew.-% |
| Wasser | 40,00 Gew.-% |
| Dimethylether | 34,96 Gew.-% |
| Parfüm, Zusatzstoffe | q.s. |

### Beispiele 127 bis 141

Pump-Haarspray-Formulierungen mit VOC-Gehalt 0:

### Polyurethan gemäß Beispielen

| | |
|---|---|
| 24-27, 29-36, 44-46 | 10,00 Gew.-% |
| Wasser | 89,97 Gew.-% |
| Parfüm, Zusatzstoffe | q.s. |

### Filmbewertung:

Alle zuvor genannten Haarspray-Formulierungen lieferten klare, feste Filme, die eine gute Flexibilität und gute Glätte aufwiesen. Die Polyurethane der Herstellungsbeispiele 28, 37, 47 und 48 mit einem hohen Siloxananteil liefern sehr weiche und leicht klebrige Filme. Diese Polyurethane sind alleine nicht als Haarfestigerpolymer geeignet, sie können jedoch als Additiv mit einem anderen Festigerpolymer zu siliconhaltigen Haarsprays mit sehr guten Eigenschaften formuliert werden. Dies wird anhand der folgenden erfindungsgemäßen Beispiele (L) bzw. Vergleichsbeispiele (VL) 1 bis 16 gezeigt.

### Beispiele (V)L1 bis 16

Die Polyurethane aus den Beispielen 37, 47 und 48 wurden einzeln und in Form von Mischungen mit konventionellen, siloxanfreien Haarfestigerpolymeren sowie gegebenenfalls unter Zusatz eines wasserunlöslichen Silicons zu 5 gew.-%igen ethanolischen Lösungen formuliert. Die Zusammensetzungen dieser Formulierungen sind in Tabelle 6 wiedergegeben. Sie wurden auf eine Glasplatte aufgetragen und die resultierenden Filme wurden im Hinblick auf 5 Kriterien, die in Tabelle 5 angegeben sind, geprüft und mit Noten von 1 bis 4 bewertet. Die Bewertungen der Filme sind in Tabelle 7 wiedergegeben.

Als Vergleichsbeispiele dienten zum einen reine 95 gew.-%ige Lösungen der aus dem Stand der Technik bekannten konventionellen, siloxanfreien Haarfestigerpolymere, die auch zur Modifizierung der erfindungsgemäß eingesetzten Siloxane verwendet wurden (VL1 bis VL3). Als Vergleichsbeispiele dienten weiterhin reine 95 gew.-%ige Lösungen der Polyurethane der Beispiele 37, 47 und 48 (VL 4 bis VL6). Als weiteres Vergleichsbeispiel diente eine 95 gew.-%ige Lösung eines Salzes aus einer einwertigen Carbonsäure und einem zweiwertigen Polydimethylsiloxandiamin (VL7), welches auch zur Modifizierung der erfindungsgemäß eingesetzten Siloxane verwendet wurde.

**Tabelle 5:**

| Bewertungskriterien | | |
|---|---|---|
| | | Note |
| A) Glätte | bremsend | 4 |
| | mäßig-glatt | 3 |
| | glatt | 2 |
| | sehr glatt | 1 |
| B) Haftung | schlecht | 4 |
| | mäßig | 3 |
| | gut | 2 |
| | sehr gut | 1 |
| C) Elastizität/Klebrigkeit | spröde/leicht klebrig | 4 |
| | weich/leicht klebrig | 3 |
| | weich - hart/leicht klebrig | 2-3 |
| | hart/nichtklebrig | 2 |
| | hart - elastisch/nichtklebrig | 1-2 |
| | elastisch/nichtklebrig | 1 |
| D) Auswaschbarkeit | schlecht | 4 |
| | mäßig | 3 |
| | gut | 2 |
| | sehr gut | 1 |
| E) Aussehen | ungleichmäßig trüb | 4 |
| | ungleichmäßig klar | 3 |
| | gleichmäßig klar | 2 |
| | gleichmäßig glänzend | 1 |

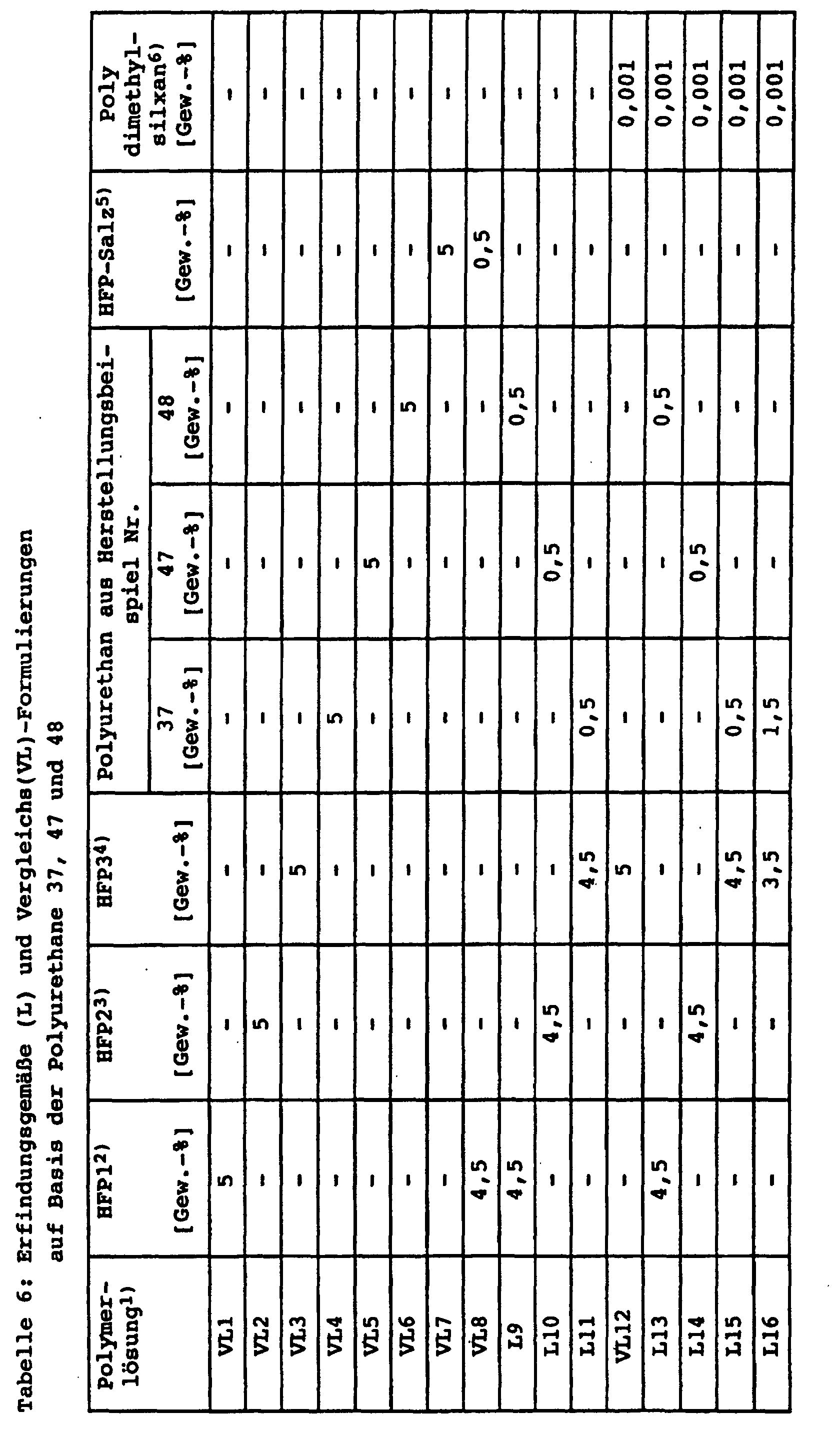

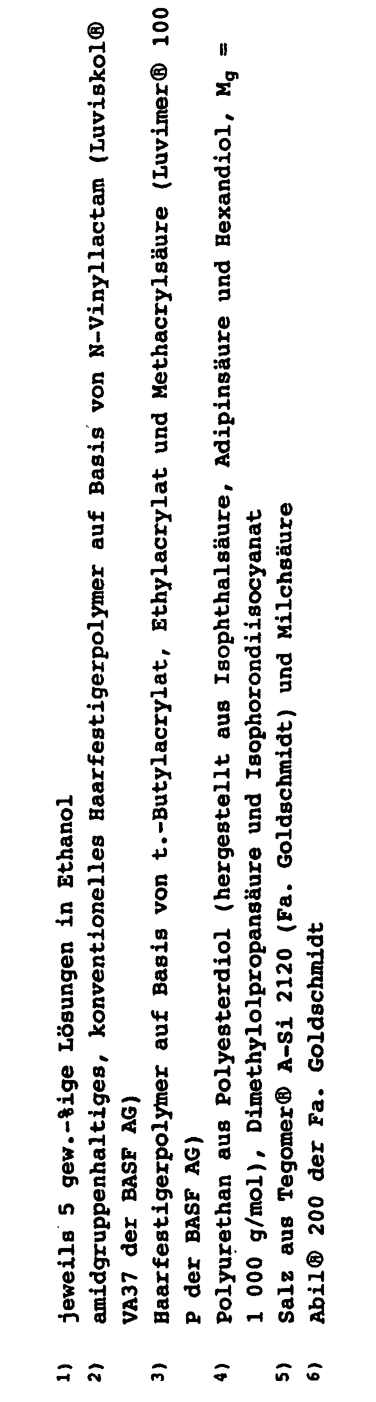

**Tabelle 7:**

| Filmeigenschaften der Polyurethane | | | | | |
|---|---|---|---|---|---|
| Polymerlösung | Bewertung | | | | |
| | A | B | C | D | E |
| VL1 | 2 | 1 | 2 | 1 | 1-2 |
| VL2 | 3 | 1 | 2 | 2 | 2 |
| VL3 | 3 | 1 | 1-2 | 2 | 2 |
| VL4 | 1 | 1 | 2-3 | 2 | 1-2 |
| VL5 | 1 | 1 | 2-3 | 2 | 1-2 |
| VL6 | 1-2 | 1 | 3 | 2 | 1-2 |
| VL7 | -- *) | -- *) | 3 | -- *) | -- *) |
| VL8 | 1-2 | 3-4 | 3 | 1 | 1-2 |
| L9 | 1-2 | 1 | 2 | 1 | 1-2 |
| L10 | 2 | 1 | 2 | 2 | 2 |
| L11 | 2 | 1 | 1-2 | 2 | 1-2 |
| VL12 | 1-2 | 1 | 2 | 2-3 | 3 |
| L13 | 1 | 1 | 2 | 1 | 1-2 |
| L14 | 1-2 | 1 | 2 | 2 | 2 |
| L15 | 1 | 1 | 1-2 | 2 | 1-2 |
| L16 | 1 | 1 | 1 | 2 | 1-2 |

| | | | | | |
|---|---|---|---|---|---|
| *) keine Filmbildung | | | | | |

## Patentansprüche

1. Kosmetisches Mittel, enthaltend wenigstens ein vernetztes, wasserlösliches oder wasserdispergierbares Polyurethan aus
A) mindestens einem Polyurethanpräpolymer mit endständigen Isocyanatgruppen und
B) mindestens einem Polymerisat mit gegenüber Isocyanatgruppen reaktiven Gruppen, die ausgewählt sind unter Hydroxyl-, primären und sekundären Amino- und/oder Carboxyl-gruppen, wobei das Polymerisat B) ausgewählt ist unter
B1) Polymerisaten, die wenigstens ein α,β-ethylenisch ungesättigtes Monomer einpolymerisiert enthalten, das zusätzlich wenigstens eine gegenüber Isocyanatgruppen reaktive Gruppe pro Molekül enthält,
B2) Polyestern,
B3) Silicon-poly(alkylenoxid)-Copolymeren,
und Mischungen davon,
wobei mindestens eine der Komponenten A) und/oder B) in Wasser löslich oder dispergierbar ist und wenigstens eine der Komponenten A) und/oder B) mindestens eine Siloxangruppe enthält,
und die Salze davon.

2. Mittel nach Anspruch 1, wobei es sich bei der Komponente A) um mindestens ein Polyurethanpräpolymer aus
a) mindestens einer Verbindung mit einem Molekulargewicht im Bereich von 56 bis 300, die zwei aktive Wasserstoffatome pro Molekül enthält,
b) mindestens einem Polymerisat mit zwei aktiven Wasserstoffatomen pro Molekül,
c) gegebenenfalls einem Polysiloxan,
d) gegebenenfalls mindestens einer Verbindung, die zwei aktive Wasserstoffatome und mindestens eine ionogene bzw. ionische Gruppe pro Molekül aufweist,
e) mindestens einem Diisocyanat
handelt.

3. Mittel nach Anspruch 2, wobei es sich bei dem Polysiloxan um eine Verbindung der Formel I 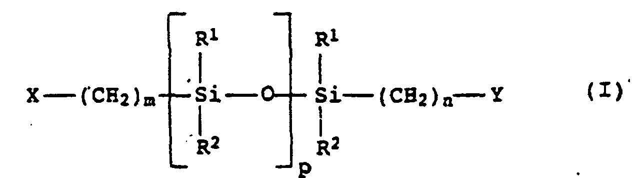 handelt, worin
R¹ und R² unabhängig voneinander für C₁- bis C₄-Alkyl, Benzyl oder Phenyl stehen,
X und Y unabhängig voneinander für OH oder NHR³ stehen, wobei R3 für Wasserstoff, C₁- bis C₆-Alkyl oder C₅- bis C₈-Cycloalkyl steht,
m und n unabhängig voneinander für 2 bis 8 stehen,
p für 3 bis 50 steht.

4. Mittel nach einem der Ansprüche 2 oder 3, wobei das Verhältnis von NCO-Äquivalent der Verbindungen der Komponente e) zu Äquivalent aktives Wasserstoffatom der Komponenten a), b), c) und d) in einem Bereich von 1,01:1 bis 1,4:1, bevorzugt 1,03:1 bis 1,3:1, insbesondere 1,05:1 bis 1,25:1, liegt.

5. Mittel nach Anspruch 1, wobei das Polymerisat B1)
f) wenigstens ein α,β-ethylenisch ungesättigtes Monomer, das zusätzlich wenigstens eine gegenüber Isocyanatgruppen reaktive Gruppe pro Molekül enthält,
g) gegebenenfalls wenigstens ein α,β-ethylenisch ungesättigtes Monomer, das ausgewählt ist unter Estern α,β-ethylenisch ungesättigter Mono- und/oder Dicarbonsäuren mit C₁bis C₂₂-Alkanolen, Amiden α,β-ethylenisch ungesättigter Mono- und/oder Dicarbonsäuren mit Mono- und Di-C₁- bis C₂₂-alkylaminen, Estern von Vinylalkohol und Allylalkohol mit C₁- bis C₄₀-Monocarbonsäuren, Vinylethern, Vinylaromaten, Vinylhalogeniden, Vinylidenhalogeniden C₂- bis C₈-Monoolefinen, nichtaromatischen Kohlenwasserstoffen mit mindestens 2 konjugierten Doppelbindungen und Mischungen davon,
h) gegebenenfalls wenigstens ein α,β-ethylenisch ungesättigtes Monomer, das ausgewählt ist unter N-Vinylamiden, N-Vinyllactamen, primären Amiden α,β-ethylenisch ungesättigter Monocarbonsäuren, vinyl- und allylsubstituierten heteroaromatischen Verbindungen und Mischungen davon,
i) gegebenenfalls wenigstens ein weiteres Monomer mit einer radikalisch polymerisierbaren, α,β-ethylenisch ungesättigten Doppelbindung und mindestens einer ionogenen bzw. ionischen Gruppe pro Molekül,
einpolymerisiert enthält.

6. Mittel nach Anspruch 1, wobei es sich bei dem Polymerisat B2) um einen Polyester auf Basis eines Polyesterdiols mit einem zahlenmittleren Molekulargewicht von 500 bis 1000 und einer aromatischen Di- oder Polycarbonsäure oder eines Carbonsäureanhydrids davon, bevorzugt Trimellithsäureanhydrid, handelt.

7. Mittel nach Anspruch 1, wobei es sich bei dem Polymerisat B3) um eine Verbindung der allgemeinen Formel II handelt, 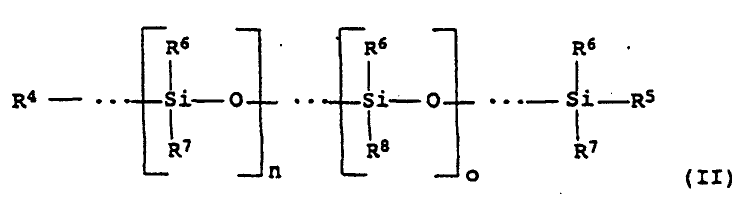 worin
n und o unabhängig voneinander für eine ganze Zahl von 0 bis 200 stehen, wobei die Summe aus n und o ≥ 3 ist,
R⁴, R⁵ und R⁸ unabhängig voneinander für C₁- bis C₈-Alkyl, Benzyl, Phenyl oder einen Rest der Formel III
-(CH₂)ᵣ-O-(CH₂CH₂O)ₚ(CH₂CH(CH₃)O)_{q}-H (III)
stehen,
wobei in der Formel III die Reihenfolge der Alkylenoxid einheiten beliebig ist,
r für eine ganze Zahl von 1 bis 8 steht,
p und q unabhängig voneinander für eine ganze Zahl von 0 bis 200 stehen, wobei die Summe aus p und q > 0 ist,
R⁶ und R⁷ unabhängig voneinander für C₁- bis C₈-Alkyl, Benzyl oder Phenyl stehen,
wobei die Verbindung der Formel II mindestens zwei Reste der allgemeinen Formel III umfasst.

8. Mittel nach einem der vorhergehenden Ansprüche, wobei das Verhältnis von NCO-Äquivalent der Komponente A) zu Äquivalent aktives Wasserstoffatom der Komponente B) in einem Bereich von 20:1 bis 1:1, bevorzugt 10:1 bis 1:1, liegt.

9. Mittel nach einem der vorhergehenden Ansprüche, wobei die Polyurethane einen Anteil an siloxanhaltigen Verbindungen der Formel I und/oder II, bezogen auf das Gesamtgewicht der eingebauten Komponenten, von 0,05 bis 30 Gew.-%, bevorzugt 0,05 bis 25 Gew.-%, insbesondere 0,05 bis 15 Gew.-%, aufweisen.

10. Mittel nach Anspruch 9, enthaltend wenigstens ein Polyurethan mit einem Siloxangehalt im Bereich von 5 bis 20 Gew.-%., bevorzugt 7 bis 17 Gew.-%, als Lösungsvermittler für hydrophobe Produkte, insbesondere Silicone, und als Additive für Haarbehandlungsmittel.

11. Mittel nach Anspruch 9, enthaltend wenigstens ein Polyurethan mit einem Siloxangehalt im Bereich von 0,05 bis 15 Gew.-%, in Form eines Haarbehandlungsmittels, insbesondere in Form eines Haarsprays.

12. Mittel nach Anspruch 11, enthaltend
a) 0,5 bis 20 Gew.-% mindestens eines in Wasser löslichen oder dispergierbaren Polyurethans, wie in einem der Ansprüche 1 bis 9 definiert,
b) 40 bis 99 Gew.-%, bevorzugt 50 bis 98 Gew.-%, eines Lösungsmittels, ausgewählt unter Wasser und wassermischbares Lösungsmitteln, bevorzugt C₂- bis C₅-Alkoholen, insbesondere Ethanol, und Mischungen davon,
c) 0 bis 50 Gew.-% eines Treibmittels, vorzugsweise Dimethylether,
d) 0 bis 15 Gew.-% mindestens eines von a) verschiedenen, in Wasser löslichen oder dispergierbaren Haarpolymers,
e) 0 bis 0,2 Gew.-% mindestens eines wasserunlöslichen Silicons,
f) 0 bis 2 Gew.-% mindestens eines nichtionischen, siloxanhaltigen, in Wasser löslichen oder dispergierbaren Polymers.

13. Verwendung der Polyurethane, wie in einem der Ansprüche 1 bis 9 definiert, als Hilfsmittel in der Kosmetik, bevorzugt als oder in Beschiehtungsmittel(n) für Haar, Haut und Nägel, insbesondere in der Haarkosmetik, als Hilfsmittel in der Pharmazie, bevorzugt als oder in Beschichtungsmittel(n) oder Bindemittel(n) für feste Arzneiformen, sowie als oder in Beschichtungsmittel(n) für die Textil-, Papier-, Druck-, Leder- und Klebstoffindustrie.

## Claims

1. A cosmetic composition comprising at least one crosslinked, water-soluble or water-dispersible polyurethane formed from
A) at least one polyurethane prepolymer having terminal isocyanate groups, and
B) at least one polymer having isocyanate-reactive groups selected from hydroxyl, primary and secondary amino and/or carboxyl groups, where the polymer B) is selected from
B1) polymers comprising in copolymerized form at least one α,β-ethylenically unsaturated monomer which additionally comprises at least one isocyanate-reactive group per molecule,
B2) polyesters,
B3) silicon-poly(alkylene oxide) copolymers,
and mixtures thereof,
at least one of components A) and/or B) being soluble or dispersible in water and at least one of components A) and/or B) comprising at least one siloxane group,
and the salts thereof.

2. A composition as claimed in claim 1, where component A) is at least one polyurethane prepolymer formed from
a) at least one compound having a molecular weight in the range from 56 to 300 which comprises two active hydrogen atoms per molecule,
b) at least one polymer having two active hydrogen atoms per molecule,
c) if desired, a polysiloxane,
d) if desired, at least one compound which has two active hydrogen atoms and at least one ionogenic or ionic group per molecule,
e) at least one diisocyanate.

3. A composition as claimed in claim 2, where the polysiloxane is a compound of the formula I 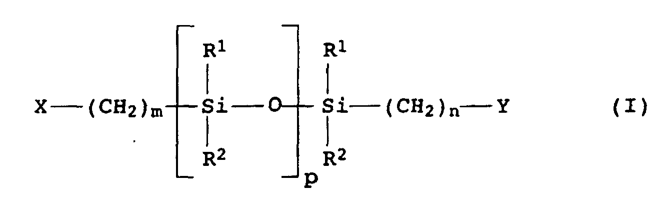 where
R¹ and R² independently of one another are C₁- to C₄-alkyl, benzyl or phenyl,
X and Y independently of one another are OH or NHR³, where R³ is hydrogen, C₁- to C₆-alkyl or C₅- to C₈-cycloalkyl,
m and n independently of one another are from 2 to 8, and
p is from 3 to 50.

4. A composition as claimed in either of claims 2 and 3, where the ratio of NCO equivalent of the compounds of component e) to equivalent of active hydrogen atom of components a), b), c) and d) lies within a range from 1.01:1 to 1.4:1, preferably from 1.03:1 to 1.3:1 and, in particular, from 1.05:1 to 1.25:1.

5. A composition as claimed in claim 1, where the polymer B1) comprises
f) at least one α,β-ethylenically unsaturated monomer which additionally comprises at least one isocyanate-reactive group per molecule,
g) if desired, at least one α,β-ethylenically unsaturated monomer selected from esters of α,β-ethylenically unsaturated mono- and/or dicarboxylic acids with C₁-C₂₂-alkanols, amides of α,β-ethylenically unsaturated mono and/or dicarboxylic acids with mono- and di-C₁-C₂₂-alkylamines, esters of vinyl alcohol and allyl alcohol with C₁-C₄₀ monocarboxylic acids, vinyl ethers, vinylaromatic compounds, vinyl halides, vinylidene halides, C₂-C₈ monoolefins, nonaromatic hydrocarbons having at least two conjugated double bonds, and mixtures thereof,
h) if desired, at least one α,β-ethylenically unsaturated monomer selected from N-vinylamides, N-vinyllactams, primary amides of α,β-ethylenically unsaturated monocarboxylic acids, vinyl- and allyl-substituted heteroaromatic compounds, and mixtures thereof,
i) if desired, at least one further monomer having a free-radically polymerizable α,β-ethylenically unsaturated double bond and at least one ionogenic and/or ionic group per molecule
in copolymerized form.

6. A composition as claimed in claim 1, where the polymer B2) is a polyester based on a polyesterdiol having a number-average molecular weight of from 500 to 1000 and on an aromatic di-or polycarboxylic acid or an anhydride thereof, preferably trimellitic anhydride.

7. A composition as claimed in claim 1, where the polymer B3) is a compound of the formula II 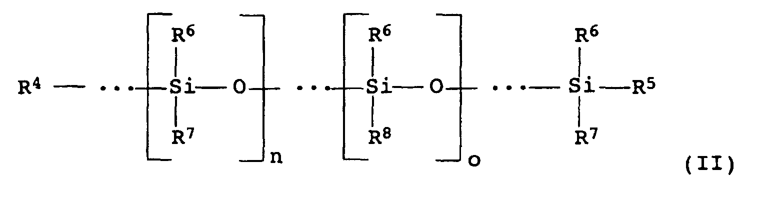 where
n and o independently of one another are an integer from 0 to 200, the sum of n and o being ≥ 3,
R⁴, R⁵ and R⁸ independently of one another are C₁-C₈-alkyl, benzyl, phenyl or a radical of the formula III
-(CH₂)ᵣ-O-(CH₂CH₂O)ₚ(CH₂CH(CH₃)O)_{q}-H (III)
in which the sequence of the alkylene oxide units is arbitrary,
r is an integer from 1 to 8,
p and q independently of one another are an integer from 0 to 200, the sum of p and q being > 0,
R⁶ and R⁷ independently of one another are C₁-C₈-alkyl, benzyl or phenyl,
and the compound of the formula II includes at least two radicals of the formula III.

8. A composition as claimed in any one of the preceding claims, where the ratio of NCO equivalent of component A) to equivalent of active hydrogen atom of component B) lies within a range from 20:1 to 1:1, preferably from 10:1 to 1:1.

9. A composition as claimed in any one of the preceding claims, where the polyurethanes have a proportion of siloxane-containing compounds of the formula I and/or II, based on the overall weight of the incorporated components, of from 0.05 to 30% by weight, preferably from 0.05 to 25% by weight and, in particular, from 0.05 to 15% by weight.

10. A composition as claimed in claim 9, comprising at least one polyurethane having a siloxane content in the range from 5 to 20% by weight, preferably from 7 to 17% by weight, as a solubilizer for hydrophobic products, especially silicones, or as an additive for hair treatment compositions.

11. A composition as claimed in claim 9, comprising at least one polyurethane having a siloxane content in the range from 0.05 to 15% by weight, in the form of a hair treatment composition, especially in the form of a hairspray.

12. A composition as claimed in claim 11 comprising
a) from 0.5 to 20% by weight of at least one polyurethane which is dispersible or soluble in water, as defined in any of claims 1 to 9,
b) from 40 to 99% by weight, preferably from 50 to 98% by weight, of a solvent selected from water and water-miscible solvents, preferably C₂ to C₅ alcohols, especially ethanol, and mixtures thereof,
c) from 0 to 50% by weight of a propellant, preferably dimethyl ether,
d) from 0 to 15% by weight of at least one hair polymer which is different from a) and is dispersible or soluble in water,
e) from 0 to 0.2% by weight of at least one water-insoluble silicone,
f) from 0 to 2% by weight of at least one nonionic, siloxane-containing polymer which is dispersible or soluble in water.

13. The use of a polyurethane as defined in any of claims 1 to 9 as an auxiliary in cosmetics, preferably as or in coating compositions for hair, skin and nails, especially in hair cosmetics, as an auxiliary in pharmacy, preferably as or in coating compositions or binders for solid drug forms, and as or in coating compositions for the textile, paper, printing, leather and adhesives industry.

## Revendications

1. Produit cosmétique, contenant au moins un polyuréthanne réticulé, hydrosoluble ou dispersable dans l'eau, émanant de
A) au moins un prépolymère de polyuréthanne ayant des groupes isocyanate terminaux et
B) au moins un produit de polymérisation ayant des groupes réactifs vis-à-vis des groupes isocyanate, qui sont choisis parmi les groupes hydroxyle, amino primaire et secondaire et/ou carboxyle, tandis que le produit de polymérisation B) est choisi parmi
B1) les produits de polymérisation qui contiennent à l'état copolymérisé au moins un monomère α,β-éthylénique-ment insaturé, qui contient en outre au moins un groupe réactif vis-à-vis des groupes isocyanate par molécule,
B2) les polyesters,
B3) les copolymères de silicone-poly(oxyde d'alkylène), et leurs mélanges,
tandis qu'au moins l'un des composants A) et/ou B) est soluble ou dispersable dans l'eau et qu'au moins l'un des composants A) et/ou B) contient au moins un groupe siloxane,
et leurs sels.

2. Produit selon la revendication 1, dans lequel il s'agit pour le composant A) d'au moins un prépolymère de polyuréthanne émanant de
a) au moins un composé ayant une masse moléculaire dans l'intervalle de 56 à 300, qui contient deux atomes d'hydrogène actif par molécule,
b) au moins un produit de polymérisation ayant deux atomes d'hydrogène actif par molécule,
c) éventuellement un polysiloxane,
d) éventuellement au moins un composé qui présente deux atomes d'hydrogène actif et au moins un groupe respectivement ionogène ou ionique par molécule,
e) au moins un diisocyanate.

3. Produit selon la revendication 2, dans lequel il s'agit pour le polysiloxane d'un composé de formule I 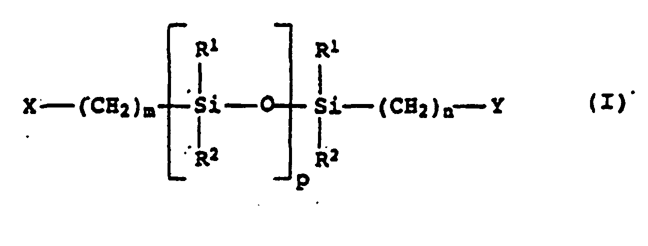 où
R¹ et R² désignent indépendamment l'un de l'autre un alkyle en C₁ à C₄, un benzyle ou un phényle,
X et Y représentent indépendamment l'un de l'autre OH ou NHR³, tandis que R³ désigne l'hydrogène, un alkyle en C₁ à C₆ ou un cycloalkyle en C₅ à C₈,
m et n désignent indépendamment l'un de l'autre 2 à 8,
p vaut de 3 à 50.

4. Produit selon l'une des revendications 2 ou 3, dans lequel le rapport d'équivalents NCO des composés du composant e) aux équivalents d'atome d'hydrogène actif des composants a), b), c) et d) se situe dans l'intervalle de 1,01:1 à 1,4:1, de préférence de 1,03:1 à 1,3:1, en particulier de 1,05:1 à 1,25:1.

5. Produit selon la revendication 1, dans lequel le produit de polymérisation B1) contient à l'état copolymérisé
f) au moins un monomère α,β-éthyléniquement insaturé, qui contient en outre au moins un groupe réactif vis-à-vis des groupes isocyanate par molécule,
g) éventuellement au moins un monomère α,β-éthyléniquement insaturé, qui est choisi parmi les esters de mono- et/ ou diacides carboxyliques α,β-éthyléniquement insaturés avec des alcanols en C₁ à C₂₂, les amides d'e mono- ou diacides carboxyliques α,β-éthyléniquement insaturés avec des mono- et dialkylamines en C₁ à C₂₂, les esters d'alcool vinylique et d'alcool allylique avec des monoacides carboxyliques en C₁ à C₄₀, les éthers vinyliques, les composés aromatiques vinyliques, les halogénures de vinyle, les halogénures de vinylidène de monooléfines en C₂ à C₈, les hydrocarbures non-aromatiques ayant au moins 2 doubles liaisons conjuguées, et leurs mélanges,
h) éventuellement au moins un monomère α,β-éthyléniquement insaturé, qui est choisi parmi les N-vinylamides, les N-vinyllactames, les amides primaires de monoacides carboxyliques α,β-éthyléniquement insaturés, les composés hétéroaromatiques vinyl- et allylsubstitués, et leurs mélanges,
i) éventuellement au moins un autre monomère ayant une double liaison α,β-éthyléniquement insaturée, polymérisable par voie radicalaire, et au moins un groupe respectivement ionogène ou ionique par molécule.

6. Produit selon la revendication 1, dans lequel il s'agit pour le produit de polymérisation B2) d'un polyester à base d'un polyesterdiol ayant une masse moléculaire moyenne en nombre de 500 à 1000 et d'un di- ou polyacide carboxylique aromatique ou d'un anhydride d'acide carboxylique de celui-ci, de préférence d'anhydride d'acide trimellitique.

7. Produit selon la revendication 1, dans lequel il s'agit pour le produit de polymérisation B3) d'un composé de formule générale II 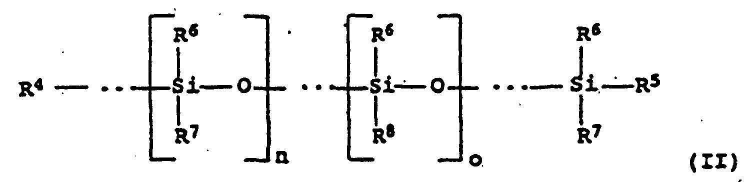 où
n et o désignent indépendamment l'un de l'autre un nombre entier de 0 à 200, tandis que la somme de n et o est ≥ 3,
R⁴, R⁵ et R⁸ représentent indépendamment l'un de l'autre un alkyle en C₁ à C₈, un benzyle, un phényle ou un reste de formule III
-(CH₂)ᵣ-O-(CH₂CH₂O)ₚ(CH₂CH(CH₃)O)_{q}-H (III)
tandis que dans le formule III la succession des unités oxyde d'alkylène est quelconque,
r désigne un nombre entier de 1 à 8,
p et q désignent indépendamment l'un de l'autre un nombre entier de 0 à 200, tandis que la sommede p et q est > 0,
R⁶ et R⁷ désignent indépendamment l'un de l'autre un alkyle en C₁ à C₈, un benzyle ou un phényle,
tandis que le composé de formule II possède au moins deux restes de formule générale III.

8. Produit selon l'une des revendications précédentes, dans lequel le rapport d'équivalents NCO du composant A) aux équivalents d'atomes d'hydrogène actif du composant B) se situe dans l'intervalle de 20:1 à 1:1, de préférence de 10:1 à 1:1.

9. Produit, selon l'une des revendications précédentes, dans lequel les polyuréthannes présentent une fraction de composés contenant du siloxane de formule I et/ou II, par rapport à la masse totale des composants incorporés, de 0,05 à 30 % en poids, de préférence de 0,05 à 25 % en poids, en particulier de 0,05 à 15 % en poids.

10. Produit selon la revendication 9, contenant au moins un polyuréthanne ayant une teneur en siloxane dans l'intervalle de 5 à 20 % en poids, de préférence de 7 à 17 % en poids, comme adjuvants de dissolution pour des produits hydrophobes, en particulier des silicones, et comme additifs pour des produits de traitement capillaire.

11. Produit selon la revendication 9, contenant au moins un polyuréthanne ayant une teneur en siloxane dans l'intervalle de 0,05 à 15 % en poids, de préférence de 7 à 17 % en poids, sous forme d'un produit de traitement capillaire, en particulier sous forme d'un spray capillaire.

12. Produit selon la revendication 11, contenant
a) 0,5 à 20 % en poids d'au moins un polyuréthanne soluble ou dispersable dans l'eau, tel que défini dans l'une des revendications 1 à 9,
b) 40 à 99 % en poids, de préférence 50 à 98 % en poids, d'un solvant, choisi parmi l'eau et les solvants miscibles avec l'eau, de préférence les alcools en C₂ à C₅, en particulier l'éthanol, et leurs mélanges,
c) 0 à 50 % en poids d'un agent propulseur, de préférence l'éther diméthylique,
d) 0 à 15 % en poids d'au moins un polymère capillaire différent de a), soluble ou dispersable dans l'eau,
e) 0 à 0,2 % en poids d'au moins un silicone hydrosoluble,
f) 0 à 2 % en poids d'au moins un polymère non-ionique, contenant du siloxane, soluble ou dispersable dans l'eau.

13. Utilisation des polyuréthannes tels que définis dans l'une des revendications 1 à 9, comme adjuvants en cosmétique, de préférence comme ou dans un(des) produit(s) d'enduction pour les cheveux, la peau ou les ongles, en particulier en cosmétique capillaire, comme adjuvants en pharmacie, de préférence comme ou dans un(des) produit(s) d'enduction ou un(des) liant(s) pour formes médicales solides, ainsi que comme ou dans un(des) produit(s) d'enduction pour l'industrie du textile, du papier, de l'imprimerie, du cuir et des colles et adhésifs.
